# EUROPEAN PATENT SPECIFICATION

(11) **EP 1 549 615 B1**
(45) Date of publication and mention of the grant of the patent: **03.12.2008**
(21) Application number: 03791529.5
(22) Date of filing: 26.08.2003
(51) Int. Cl.: C07D 211/26, A61K 31/451, A61P 25/00, A61P 9/00

(54) **NAPHTHAMIDE DERIVATIVES AND THEIR USE**
NAPHTHAMIDDERIVATE UND DEREN VERWENDUNG
DERIVES DE NAPHTAMIDE ET LEUR UTILISATION

(30) Priority: 29.08.2002 SE 0202567; 09.10.2002 SE 0202986
(43) Date of publication of application: 06.07.2005
(73) Proprietor: AstraZeneca AB, 151 85 Södertälje (SE)
(72) Inventor: BERNSTEIN, Peter AstraZeneca Wilmington, Wilmington, DE 19850-5437 (US); DANTZMAN, Cathy AstraZeneca Wilmington, Wilmington, DE 19850-5437 (US); DEDINAS, Robert AstraZeneca Wilmington, Wilmington, DE 19850-5437 (US); SHEN, Lihong AstraZeneca Wilmington, Wilmington, DE 19850-5437 (US); WARWICK, Paul AstraZeneca Wilmington, Wilmington, DE 19850-5437 (US)
(74) Representative: Hallybone, Huw George
(86) International application number: PCT/SE2003/001329
(87) International publication number: WO 2004/020411

(56) References cited:
- WO-A1-00/02859
- WO-A1-00/20389
- WO-A1-00/25786
- WO-A1-94/13639
- WO-A1-02/051807
- THOMAS RYCKMANS ET AL.: 'First Dual NK1 Antagonists-Serotonin Reuptake Inhibitors: Synthesis and SAR of a New Class of Potential Antidepressants' BIOORGANIC & MEDICINAL CHEMISTRY LETTERS vol. 12, no. 2, January 2002, pages 261 - 264, XP002974383

## Description

### FIELD OF THE INVENTION

This invention relates to the treatment of diseases in which serotonin and Substance P or Neurokinin A are implicated, for example, in the treatment of disorders or conditions such as hypertension, depression, generalized anxiety disorder, phobias, posttraumatic stress syndrome, avoidant personality disorder, premature ejaculation, eating disorders, obesity, chemical dependencies, cluster headache, migraine, pain, Alzheimer's disease, obsessive-compulsive disorder, panic disorder, memory disorders, Parkinson's disease, endocrine disorders vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania and headache.

### BACKGROUND

The mammalian neurokinins are peptide neurotransmitters found in the peripheral and central nervous systems. The three principal neurokinins are Substance P (SP), Neurokinin A (NKA) and Neurokinin B (NKB). N-terminally extended forms of at least NKA are known. Three receptor types are known for the principal neurokinins. Based upon their relative selectivities for the neurokinins SP, NKA and NKB, the receptors are classified as neurokinin 1 (NK₁), neurokinin 2 (NK₂) and neurokinin 3 (NK₃) receptors, respectively. In the periphery, SP and NKA are localized in C-afferent sensory neurons, which neurons are characterized by non-myelinated nerve endings known as C-fibers, and are released by selective depolarization of these neurons, or selective stimulation of the C-fibers. C-Fibers are located in the airway epithelium, and the tachykinins are known to cause profound effects which clearly parallel many of the symptoms observed in asthmatics. The effects of release or introduction of tachykinins in mammalian airways include bronchoconstriction, increased microvascular permeability, vasodilation, increased mucus secretion and activation of mast cells. Neurokinin antagonists that interact with NK₁, NK₂ and NK₃ receptors, having different chemical structures have been described.

NK₁ activity is also implicated in depression and anxiety, mice with genetically altered NK₁ receptors have decreased anxiety related behavior (Santarelli, L., et al., Proc. Nat. Acad. Sci. (2001), 98, 1912) and NK₁ antagonists have been reported to be effective in an animal model of depression (Papp, M., et al., Behav. Brain Res. (2000), 115, 19).

Serotonin Selective Reuptake Inhibitors (SSRIs) are widely used for the treatment of major depressive disorder (MDD) and are considered well-tolerated and easily administered. SSRIs, however, have a delayed onset of action, are associated with undesirable side effects, such sexual dysfunction, and are ineffective in perhaps 30% of patients (M. J. Gitlin, MJ, J. Clin. Psych., 55, 406-413, 1994).

WO94/13639 discloses phenylpiperidine compounds which are tachykinin antagonists.

WO00/25786 discloses a class of heterocyclic compounds that are useful as potassium channel inhibitors to treat autoimmune disorders, cardiac arrhythmias, and the like.

WO00/02859 discloses N-substituted naphthalene carboxamides which are antagonists of at least one tachykinin receptor and are useful in the treatment of depression, anxiety, asthma, pain, inflammation, urinary incontinence and other disease conditions. Processes for their preparation are described, as are compositions containing them and their use.

WO02/051807 discloses N-substituted amides useful in the treatment of diseases and pharmaceutical composition comprising such compounds.

WO00/20389 discloses peptide neurotransmitters useful for treating depression, anxiety, asthma, rheumatoid arthritis, Alzheimer's disease, cancer, schizophrenia, oedema, allergic rhinitis, inflammation, pain, gastrointestinal-hypermotility, anxiety, emesis, Huntington's disease, psychoses including depression, hypertension, migraine, bladder hypermotility, or urticaria, along with methods of making the compounds and pharmaceutical compositions containing the compounds.

Thomas Ryckmans et al.: Bioorganic & Medicinal Chemistry Letters vol. 12, no. 2, January 2002, pages 261 - 264 discloses dual NK₁ antagonists-serotonin reuptake inhibitors as a class of potential antidepressants.

Compounds with dual action as NK₁ antagonists and serotonin reuptake inhibitors may, therefore provide a new class of antidepressants. Indeed, compounds combining NK₁ antagonism and serotonin reuptake inhibition have been described (Ryckmans, T., et al., Bioorg. Med. Chem. Lett. (2002), 12, 261).

### SUMMARY OF THE INVENTION

We have discovered naphthamide derivatives having both neurokinin 1 (NK₁) antagonist activity and serotonin reuptake inhibitory (SRI) activity. Naphthamide derivatives of the invention are compounds in accord with structural diagram I: 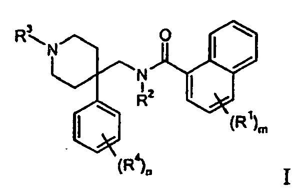 wherein:
R¹ independently at each occurrence is CN, CF₃,OCF₃, OCHF₂, halogen, C₂₋₄alkenyl, C₂₋₄alkynyl, R^{a}, R^{b}, SR^{a}, NR^{a}R^{b}, CH₂NR^{a}R^{b}, OR^{a} or CH₂OR^{a}, where R^{a} and R^{b} are independently at each occurrence hydrogen, C₁₋₆alkyl, C(O)R^{c}, C(O)NHR^{c} or CO₂R^{c}, where R^{c} at each occurrence is C₁₋₆alkyl; or, R^{a} and R^{b} together are (CH₂)jG(CH₂)ₖ or G(CH₂)ⱼG, where G is oxygen or sulfur, j is 1, 2, 3 or 4, and k is 0, 1 or 2;
m is 1, 2 or 3 where at least one R¹ moiety is other than hydrogen;
R² and R³ are independently hydrogen, C₁₋₆alkyl or C₁₋₆alkyl substituted with C₁₋₄alkoxy;
R⁴ independently at each occurrence is hydrogen, CN, CF₃,OCF₃, OCHF₂, halogen, C₁₋₄alkyl, C₂₋₄alkenyl, C₂₋₄alkynyl, SR^{a}, NR^{a}R^{b}, CH₂NR^{a}R^{b}, OR^{a} or CH₂OR^{a}, where R^{a} and R^{b} are independently at each occurrence hydrogen, C₁₋₆alkyl, C(O)R^{c}, C(O)NHR^{c} or CO₂R^{c} where R^{c} at each occurrence is C₁₋₆alkyl; or, R^{a} and R^{b} together are (CH₂)jG(CH₂)k or G(CH₂)ⱼG where G is oxygen or sulfur, j is 1, 2, 3 or 4, k is 0, 1 or 2, and
n is 0, 1, 2 or 3.

The invention also encompasses pharmaceutically-acceptable salts of the naphthamide derivatives, pharmaceutical compositions and formulations containing them, processes and intermediates used to prepare them, uses of them as medicaments, uses of them in the manufacture of medicaments and uses of them for diagnostic and analytic purposes.

### DETAILED DESCRIPTION OF THE INVENTION i

Compounds of the present invention are those in accord with structural diagram I: 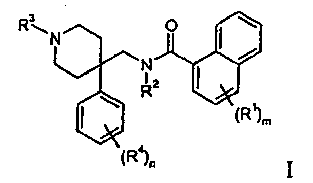 wherein:
R¹ at each occurrence is independently selected from CN, CF₃,OCF₃, OCHF₂, halogen, C₂₋₄alkenyl, C₂₋₄alkynyl, R^{a}, R^{b}, SR^{a}, NR^{a}R^{b}, CH₂NR^{a}R^{b}, OR^{a} or CH₂OR^{a}, where R^{a} and R^{b} are independently at each occurrence hydrogen, C₁₋₆alkyl, C(O)R^{c}, C(O)NHR^{c} or CO₂R^{c}, where R^{c} at each occurrence is C₁₋₆alkyl; or, R^{a} and R^{b} together are (CH₂)jG(CH₂)ₖ or G(CH₂)ⱼG, where G is oxygen or sulfur, j is 1, 2, 3 or 4, and k is 0, 1 or 2;
m is 1, 2 or 3 where at least one R¹ moiety is other than hydrogen;
R² and R³ are independently hydrogen, C₁₋₆alkyl or C₁₋₆alkyl substituted with C₁₋₄alkoxy;
R⁴ at each occurrence is independently selected from hydrogen, CN, CF_{3,}OCF₃, OCHF₂, halogen, C₁₋₄alkyl, C₂₋₄alkenyl, C₂₋₄alkynyl, SR^{a}, NR^{a}R^{b}, CH₂NR^{a}R^{b}, OR^{a} or CH₂OR^{a}, where R^{a} and R^{b} are independently at each occurrence hydrogen, C₁₋₆alkyl, C(O)R^{c}, C(O)NHR^{c} or CO₂R^{c} where R^{c} at each occurrence is C₁₋₆alkyl; or, R^{a} and R^{b} together are (CH₂)jG(CH₂)k or G(CH₂)ⱼG, and
n is 0, 1, 2 or 3;
and pharmaceutically-acceptable salts thereof.

Other particular compound of the invention are those wherein:
R¹ independently at each occurrence is CN, C₁₋₆alkyl or OR^{c} and m is 1, 2 or 3;
R² and R³ are independently hydrogen or C₁₋₆alkyl, and
R⁴ independently at each occurrence is halogen where n is 1 or 2;
and pharmaceutically-acceptable salts thereof.

More particular compound of the invention are those wherein:
R¹ independently at each occurrence is CN, ethyl or methoxy and m is 1, 2 or 3;
R² and R³ are independently hydrogen or methyl, and
R⁴ independently at each occurrence is halogen where n is 1 or 2;
and pharmaceutically-acceptable salts thereof.

Particular compounds of the invention are those wherein Ar, R¹, R² and R³ are moieties identified in Table 2 and Table 3, herein.

Particular compounds of the invention are those according to structural diagram II 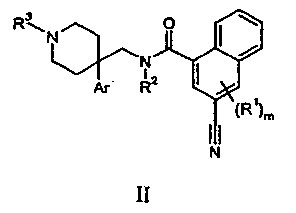 wherein Ar is selected from phenyl, 3,4-dichlorophenyl, 3-fluorophenyl, 4-fluorophenyl 3,4-difluorophenyl, 4-methoxyphenyl, 3,4-dimethoxyphenyl, 3,4-methylenedioxyphenyl, 4-difluoromethoxyphenyl or 4-trifluoromethoxyphenyl; R¹ is selected from H, methyl, ethyl or methoxy where m is 1 or 2 and R² and R³ are independently is selected from H or methyl, and in vivo-hydrolysable precursors thereof, and pharmaceutically-acceptable salts thereof.

Most particular compounds of the invention are those described herein and pharmaceutically-acceptable salts thereof.

Pharmaceutically-acceptable salts of compounds in accord with structural diagram I include those made with inorganic or organic acids which afford a physiologically-acceptable anion, such as with, for example, hydrochloric, hydrobromic, sulfuric, phosphoric, methanesulfonic, sulfamic, para-toluenesulfonic, acetic, citric, lactic, tartaric, malonic, fumaric, ethanesulfonic, benzenesulfonic, cyclohexylsulfamic, salicyclic and quinic acids.

In order to use a compound in accord with structural diagram I or a pharmaceutically-acceptable salt thereof for the therapeutic treatment or prophylactic treatment of mammals including humans, it is normally formulated in accordance with standard pharmaceutical practice as a pharmaceutical composition.

Therefore, another aspect the present invention is a pharmaceutical composition comprising a compound in accord with structural diagram I, or a pharmaceutically-acceptable salt thereof and a pharmaceutically-acceptable carrier.

Pharmaceutical compositions of this invention may be administered in standard manner for the disease condition that it is desired to treat, for example by oral, topical, parenteral, buccal, nasal, vaginal or rectal administration or by inhalation or insufflation. For these purposes the compounds of this invention may be formulated by means known in the art into the form of, for example, tablets, capsules, aq. or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aq. or oily solutions or suspensions or sterile emulsions.

In addition to the compounds of the present invention the pharmaceutical composition of this invention may also contain, or be co-administered (simultaneously or sequentially) with, one or more pharmacological agents of value in treating one or more disease conditions referred to herein.

The pharmaceutical compositions of this invention will normally be administered to humans so that, for example, a daily dose of 0.01 to 25 mg/kg body weight (and preferably of 0.1 to 5 mg/kg body weight) is received. This daily dose may be given in divided doses as necessary, the precise amount of the compound received and the route of administration depending on the weight, age and sex of the patient being treated and on the particular disease condition being treated according to principles known in the art.

Typically unit dosage forms will contain about 1 mg to 500 mg of a compound of this invention. For example a tablet or capsule for oral administration may conveniently contain up to 250 mg (and typically 5 to 100 mg) of a compound in accord with structural diagram I or a pharmaceutically-acceptable salt thereof. In another example, for administration by inhalation, a compound in accord with structural diagram I or a pharmaceutically-acceptable salt thereof may be administered in a daily dosage range of 5 to 100 mg, in a single dose or divided into two to four daily doses. In a further example, for administration by intravenous or intramuscular injection or infusion, a sterile solution or suspension containing up to 10% w/w (and typically 5% w/w) of a compound in accord with structural diagram I or a pharmaceutically-acceptable salt thereof may be used.

The compounds of the invention can be used in method of treating a disease condition wherein antagonism of NK₁ receptors in combination with SRI activity is beneficial which method comprises administering to a warm-blooded animal an effective amount of a compound in accord with structural diagram I or a pharmaceutically-acceptable salt thereof. The present invention also provides the use of a compound in accord with structural diagram I or a pharmaceutically-acceptable salt thereof in the preparation of a medicament for use in a disease condition wherein antagonism of the NK₁ receptors and SRI activity is beneficial.

The compounds of the invention can be used in a method for treating a disorder or condition selected from hypertension, depression in cancer patients, depression in Parkinson's patients, postmyocardial infarction depression, subsyndromal symptomatic depression, depression in infertile women, pediatric depression, major depression, single episode depression, recurrent depression, child abuse induced depression, post partum depression, generalized anxiety disorder, agoraphobia, social phobia, simple phobias, posttraumatic stress syndrome, avoidant personality disorder, premature ejaculation, anorexia nervosa, bulimia nervosa, obesity, addictions to alcohol, cocaine, heroin, phenobarbital, nicotine or benzodiazepines; cluster headache, migraine, pain, Alzheimer's disease, obsessive-compulsive disorder, panic disorder, dementia, amnestic disorders, age-related cognitive decline, dementia in Parkinson's disease, neuroleptic-induced parkinsonism, tardive dyskinesias, hyperprolactinaemia, vasospasm, cerebral vasculature vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania and headache associated with vascular disorders in a mammal, comprising administering an effective amount of a compound in accord with structural diagram I or a pharmaceutically-acceptable salt thereof effective in treating such disorder or condition and a pharmaceutically-acceptable carrier.

The present invention also relates to a pharmaceutical composition for treating a disorder or condition selected from hypertension, depression (e.g., depression in cancer patients, depression in Parkinson's patients, postmyocardial infarction depression, subsyndromal symptomatic depression, depression in infertile women, pediatric depression, major depression, single episode depression, recurrent depression, child abuse induced depression, and post partum depression), generalized anxiety disorder, phobias (e.g., agoraphobia, social phobia and simple phobias), posttraumatic stress syndrome, avoidant personality disorder, premature ejaculation, eating disorders (e.g., anorexia nervosa and bulimia nervosa), obesity, chemical dependencies (e.g., addictions to alcohol, cocaine, heroin, phenobarbital, nicotine and benzodiazepines), cluster headache, migraine, pain, Alzheimer's disease, obsessive-compulsive disorder, panic disorder, memory disorders (e.g., dementia, amnestic disorders, and age-related cognitive decline (ARCD)), Parkinson's diseases (e.g., dementia in Parkinson's disease, neuroteptic-induced parkinsonism and tardive dyskinesias), endocrine disorders (e.g., hyperprolactinaemia), vasospasm (particularly in the cerebral vasculature), cerebellar ataxia, gastrointestinal tract disorders (involving changes in motility and secretion), negative symptoms of schizophrenia, premenstrual syndrome, fibromyalgia syndrome, stress incontinence, Tourette's syndrome, trichotillomania, kleptomania, male impotence, attention deficit hyperactivity disorder (ADHD), chronic paroxysmal hemicrania and headache (associated with vascular disorders) in a mammal, preferably a human, comprising an effective amount of a compound in accord with structural diagram I or a pharmaceutically-acceptable salt thereof effective in treating such disorder or condition and a pharmaceutically-acceptable carrier.

Compounds in accord with structural diagram I or pharmaceutically-acceptable salts thereof may be made by processes as described and exemplified herein and by processes similar thereto and by processes known in the chemical art. If not commercially available, starting materials for these processes may be made by procedures which are selected from the chemical art using techniques which are similar or analogous to the synthesis of known compounds.

Pharmaceutically-acceptable salts may be prepared from the corresponding acid in a conventional manner. Non-pharmaceutically-acceptable salts may be useful as intermediates and as such are another aspect of the present invention.

It is well known in the art how to prepare optically-active forms (for example, by resolution of the racemic form or by synthesis from optically-active starting materials) and all optically active forms, enantiomers are compounds of this invention.

The following biological test methods, data and Examples serve to illustrate and further describe the invention.

The utility of a compound of the invention or an in vivo-hydrolysable precursor or a pharmaceutically-acceptable salt thereof (hereinafter, collectively referred to as a "Compound") may be demonstrated by standard tests and clinical studies, including those disclosed in the publications described below.

### Biological Assays:

### SERT Binding Assay:

Frozen membrane preparations of a stably transfected HEK293 cell line expressing human 5-HTT receptors were purchased from Receptor Biology (PerkinElmer). Frozen alliquots were rapidly thawed, homogenized, and diluted in assay buffer (AB) containing 50 mM TRIS-HCL, 120 mM NaCl, 5 mM KCl and adjusted to pH 7.4 with NaOH. Final protein concentration was 40 µg/ml. Test compounds were evaluated in competition assays utlilizing [³H]-Imipramine Hydrochloride purchased from NEN (PerkinElmer) as the radioligand. The stock radioligand was diluted with AB for a final concentration of approximately 2 nM. Kd for [³H]-Imipramine Hydrochloride was determined to be 2.7 nM. The competition assays were performed on 96-well assay plates - two drugs per plate. Ten serial dilutions (normally 1 µM to 38 pM final concentration) from stock 10 mM solutions of compounds prepared in DMSO. All serial dilutions were made using 20% DMSO. DMSO content in assay is less than 1%. Incubation mixtures were prepared in quadruplicate in 96-23ll plates (Costar). Final assay volumes per well were 10 µl compound/nonspecific/control (1% DMSO), 20 µl membranes, 20 µl [3H]-Imipramine Hydrochloride, and 150 µl AB. Specific binding was defined by using 10 µM Imipramine. The binding reaction was initiated by adding membranes immediately after adding the radioligand to wells containing buffer plus either test compound, nonspecific, or control. The assay plates were placed on a plate shaker and shaken for thirty minutes while the reactions reached equilibrium. The plates were then filtered through Beckman GF/B filters, presoaked in 6% PEI, using a Packard Filtermate 196. Filters were washed 5x with 0.2 ml ice-cold wash buffer (5 mM Tris HCl, pH 7.4.) After filters dried, 35 µl of Microscint20 (Packard) was added to each well. The µL of freshly made working buffer in each well. Concentrations resulting from this procedure are shown in Table 1. The final compound concentrations in the assay span 11 points, between 10 µM and 0.1 nM, in half-log increments.

**Table 1. Concentrations of compound and DMSO in various wells of a 96-well plate after serial dilution using Biomek 2000**

| Column number | Compound (Molarity) | DMSO (%) |
|---|---|---|
| 1 | 1e-4 | 1 |
| 2 | 3e-5 | 1 |
| 3 | 1e-5 | 1 |
| 4 | 3e-6 | 1 |
| 5 | 1e-6 | 1 |
| 6 | 3e-7 | 1 |
| 7 | 1e-7 | 1 |
| 8 | 3e-8 | 1 |
| 9 | 1e-8 | 1 |
| 10 | 3e-9 | 1 |
| 11 | 1e-9 | 1 |
| 12 | none | 1 |

The contents of the deep wells were mixed, and 45 µL of each dilution were transferred, in duplicate, to a 384-well polypropylene compound loading plate (Fisher 12-565-507) so that the 384-well plate contained duplicates of each of the compounds from both 96-well plates in the concentrations shown in table 1. Columns 23 & 24 of the plate contain no compound and serve as controls. Wells A - N in columns 23 and 24 were loaded with agonist only and therefore represent the maximal response. Wells O - P in columns 23 and 24 were loaded with only buffer, no agonist, and therefore represent the minimum response.

An ASMSP agonist loading plate was made by taking stock concentration of ASMSP and diluting in working buffer to give a concentration of 3.3 x 10⁻⁸ M. 45 µL of this solution were transferred to all wells of a 384-well polypropylene agonist loading plate (Fisher 12-565-507) except wells 023, 024, P23 & P24 which contained buffer alone and served as unstimulated controls.

### Dye Loading cells and adding compound:

For each 384-23ll assay plate of cells, 10 mL of diluted Fluo-4 dye was prepared as stated above in the methods/reagents section. First, each 384-well cell plate was washed once with working buffer on a CCS Packard plate washer. Any remaining post-wash buffer in the wells was removed by hand and 25 µL per well of Fluo-4 dye was added using a Labsystems Multidrop 384. The cell plate was returned to a 37 °C incubator for 45 min to allow the dye to permeate the cells. After 45 min of dye loading, the cell plates were washed twice with working buffer, leaving a 30 µL volume of buffer in each well. 5 µL of compound dilutions were transferred from the compound plate to the cell plate using a PlateMate Assay plates were incubated in the presence of compound for 15 min at room temperature in the dark, and then loaded onto FLIPR.

### Recording responses in FLIPR:

After the 15 min compound pre-incubation, the plates were loaded onto the FLIPR instrument, 15 µL of ASMSP agonist was added and the cellular response to the agonist was recorded for 90 seconds. The response is measured as the peak relative fluorescence after agonist addition.

### Data analysis:

Results contained in the *stat* files generated by FLIPR were pasted into an Excel analysis template and, after outliers were excluded, IC₅₀ values were calculated within the template using XLfit. Individual IC₅₀ values were reported, along with pIC_{50.} When the two IC₅₀'s obtained for a compound differed by more than 3-fold that compound was assayed one or two more times to re-determine the value.

Compounds of the present invention exhibit a Ki in the range of 1 to 100 nM in the SERT assay and have an IC₅₀ in the range 1 to100 nM in FLIPR assay

The invention is illustrated by, but not limited to, the following examples in which descriptions, where applicable and unless otherwise stated, the following terms, abbreviations and conditions are used:

Abbreviations used herein are as follows: aq., aqueous; atm, atmospheric pressure; BOC, 1,1-dimethylethoxycarbonyl; DCM, dichloromethane; DMF, N,N-dimethylformamide; DMSO, dimethyl sulfoxide; EtOH, ethanol; Et2O, diethyl ether; EtOAc, ethyl acetate; h, hour(s); HPLC, high pressure liquid chromatography; HOBT, 1-hydroxybenzotriazole; MeOH, methanol; min, minutes; MS, mass spectrum; NMR, nuclear magnetic resonance; psi, pounds per square inch; RT, room temperature; sat., saturated; TEA, triethylamine; TFA, trifluoroacetic acid; THF, tetrahydrofuran.

Temperatures are given in degrees Celsius (°C); unless otherwise stated, operations were carried out at room or ambient temperature (18-25 °C).

Organic solutions were dried over anhydrous sodium or magnesium sulfate; evaporation of solvent was carried out using a rotary evaporator under reduced pressure (4.5-30 mm Hg) with a bath temperature of up to 60 °C.

Chromatography means flash column chromatography on silica gel unless otherwise noted; solvent mixture compositions are given as volume percentages or volume ratios.

When given, NMR data is in the form of delta values for major diagnostic protons (given in parts per million (ppm) relative to tetramethylsilane as an internal standard) determined at 300 MHz.

Melting points are uncorrected.

Mass spectra (MS) were obtained using an automated system with atmospheric pressure chemical ionization (APCI) unless otherwise indicated. Masses corresponding to the major isotopic component, or the lowest mass for compounds with multiple masses with nearly equivalent abundance (isotope splitting), are reported.

"Halogen" or "halo," as used herein means, fluoro, chloro, bromo and iodo.

Where noted that a final compound was converted to the citrate salt, the free base was dissolved in methanol, DCM, or acetonitrile, combined with citric acid (1.0 equivalents) in methanol, concentrated under reduced pressure and dried under vacuum (25-60 °C). When indicated that the salt was isolated by filtration from Et₂O, the citrate salt of the compound was stirred in Et₂O for 4-18 h, recovered by filtration, washed with Et₂O, and dried under vacuum (25-60 °C).

### EXAMPLES

### Example 1: 1-N-Methyl-4-(3,4-dichlorophenyl)-4-(3-(3-cyanonaphth-1-yl)-(3-oxo-2-N-methyl-2-azaprop-1-yl))piperidine.

The title compound of the following structure 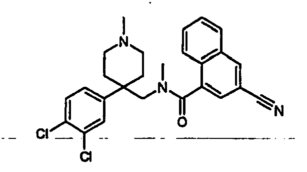 was prepared as a citrate hemihydrate, as follows. A solution containing 3-cyano-1-naphthoyl chloride (as described in US patent 6,365,602) (141.2 mg, 0.655 mmol) and dry DCM (2 mL) was added in portions (0.25 mL) to a stirred solution containing 1-N-methyl-4-(3,4-dichlorophenyl)-4-(N-methylaminomethyl)piperidine (195.5 mg, 0.681 mmol), TEA (0.13 mL), and dry DCM (5 mL) at RT. After 72h, the mixture was partitioned between DCM and 1M aq. HQAc, the organic layer was removed, and the aq. layer extracted with additional DCM (4X). The organic extracts were combined, washed (sat. aq. NaHCO₃), dried, filtered, and concentrated. The residue was purified by chromatography (2-10% MeOH-DCM w/0.5% aq. NH₃) and crystallization (DCM-hexane), converted to the citrate salt and isolated by filtration from Et₂O to give the title compound as a white powder. MS m/z 466 (M+H). Analysis for C₂₆H₂₇Cl₂N₃O. 1.0 C₆H₈O₇. 0.5 H₂O: Calculated: C, 57.58; H, 5.13; N, 6.29. Found: C, 57.42; H, 5.05; N, 6.24.

The requisite 1-N-methyl-4-(3,4-dichlorophenyl)-4-(N-methylaminomethyl)piperidine was prepared as follows:
a) 1-N-Methyl-4-(3,4-dichlorophenyl)-4-(N-methylaminomethyl)piperidine.
   A solution containing I-N-methyl-4-(3,4-dichlorophenyl)-4-(ethoxycarbonylaminomethyl) piperidine (2.14 g, 6.2 mmol) and dry THF (20 mL) was added to a LiAlH₄ and THF (40 mL) mixture at room temperature. The mixture was boiled under reflux for 1h, cooled to RT, and carefully treated with Na₂SO₄. 10 H₂O (in portions) until no further gas evolution was noted. The mixture was stirred at RT for 18h, filtered, and the solids washed with additional THF and toluene. The filtrates and washings were combined and concentrated to give the title compound as a light-yellow solid. The material was used without further purification. MS m/z 287 (M+H).
b) 1-Methyl-4-(3,4-dichlorophenyl)-4-(ethoxycarbonylaminomethyl)piperidine
   A solution containing 1-N-methyl-4-aminomethyl-4-(3,4-dichlorophenyl)piperidine (2.13 g, 7.80 mmol), TEA (1.36 mL), and dry DCM (15 mL) was cooled (ice bath), and a solution containing ethyl chloroformate (0.93 mL) and DCM (5 mL) was added dropwise over 20 min. After 40 min, cooling was removed and the solution was stirred at RT for an additional 3h. The reaction was diluted with additional DCM, washed with sat. aq. NaHCO₃ and brine, dried, filtered and concentrated. The residue was purified by chromatography (5-10% MeOH/DCM) to give the title compound as a viscous oil. MS m/z 345 (M+H).
c) 1-N-Methyl-4-aminomethyl-4-(3,4-dichlorophenyl)piperidine
   A mixture containing 1-N-methyl-4-(3,4-dichlorophenyl)-4-cyanopiperidine (2.1 g, 7.8 mmol), Raney Ni catalyst (1g of 50% aq. slurry), EtOH (50 mL), and ammonium hydroxide (25 mL) was placed under a hydrogen atmosphere (50 psi) and agitated (Parr apparatus) for 18 h. The mixture was filtered through diatomaceous earth and concentrated to give the title compound as a viscous oil that was used without further purification. MS m/z 273 (M+H).
d) 1-N-Methyl-4-(3,4-dichlorophenyl)-4-cyanopiperidine.
   According to procedures given in J. Het Chem., 20, 771 (1983); ibid., 23, 73 (1986), a mixture containing 3,4-dichlorophenylacetonitrile (4.9 g, 26.44 mmol), N-methyl-bis-(2-chloroethyl)amine hydrochloride (5.1 g, 26.49 mmol), hexadecyltributylphosphonium bromide (0.72g, 1.43 mmol), and 50% aq. sodium hydroxide (30 mL) was heated at 100 °C for 1 hour, allowed to cool, treated with water (100 mL), and extracted with Et₂O (3X). The ether extracts were combined, washed with water (1X), and extracted with 1N aq. HCl (5X). The acidic extracts were washed with Et₂O, neutralized with solid sodium carbonate, and extracted with Et₂O (2X). The ether extracts were dried, filtered and concentrated. The residual oil was purified by chromatography (0.5-2% MeOH/DCM) to give the title compound as a yellow oil. MS m/z 269 (M+H).

### Example 2: 1-N-Methyl-4-(3,4-dichlorophenyl)-4-(3-(3-cyano-2-methoxynaphth-1-yl)-(3-oxo-2-N-methyl-2-azaprop-1-yl))piperidine.

The title compound of the following structure 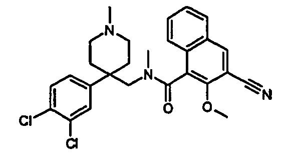 was prepared as a citrate hydrate, as follows. A solution containing 3-cyano-2-methoxy-1-naphthoyl chloride (described in international publication WO 00/20389) (151.9 mg, 0.618 mmol) and dry DCM (2 mL) was added in portions (0.25 mL) to a stirred solution containing 1-N-methyl-4-(3,4-dichlorophenyl)-4-(N-methylaminomethyl)piperidine (183.3 mg, 0.638 mmol), TEA (0.12 mL), and dry DCM (5 mL) at RT. After 72h, the mixture was partitioned between DCM and 1M aq. HOAc, the organic layer was removed, and the aq. layer extracted with additional DCM (4X). The organic extracts were combined, washed (sat. aq. NaHCO₃), dried, filtered, and concentrated. The residue was purified by chromatography (2-10% MeOH-DCM w/0.5% aq. NH₃), converted to the citrate salt and isolated by filtration from Et₂O to give the title compound (white powder) as a mixture of (E) and (Z) amides. MS m/z 496 (M+H). Analysis for C₂₇H₂₇Cl₂N₃O₂ . 1.0 C₆H₈O₇ . 1.0 H₂O: Calculated: C, 56.10; H, 5.28; N, 5.95. Found: C, 56.44; H, 5.10; N, 5.98.

### Example 3: 4-(3,4-Dichlorophenyl)-4-(3-(3-cyano-2-methoxynaphth-1-yl)-3-oxo-2-azaprop-1-yl)piperidine.

The title compound of the following structure 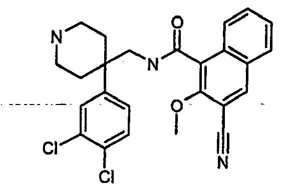 was prepared as a citrate, as follows. A solution containing 1-N-BOC-4-(3,4-dichlorophenyl)-4-(3-(3-cyano-2-methoxynaphth-1-yl)-3-oxo-2-azaprop-1-yl)piperidine (329 mg, 0.579 mmol) and DCM (5 mL) was stirred at room temperature and TFA (5 mL) was slowly added. After 18 h, the solution was concentrated, and the residue partitioned between DCM and sat. aq. NaHCO₃. The organic layer was removed and the basic aq. layer was extracted with additional DCM (2X). The organic extracts were combined, dried, filtered, and concentrated. The residue was purified by chromatography (0-5% MeOH/DCM w/0.5% aq. NH₃) and converted to the citrate salt to give the title compound as a white powder. MS m/z 468 (M+H).

The requisite 1-N-BOC-4-(3,4-dichlorophenyl)-4-(3-(3-cyano-2-methoxynaphth-1-yl)-3-oxo-2-azaprop-1-yl)piperidine was prepared as follows:
a) 1-N-BOC-4-(3,4-dichlorophenyl)-4-(3-(3-cyano-2-methoxynaphth-1-yl)-3-oxo-2-azaprop-1-yl) piperidine.
   To a stirred solution containing 1-N-BOC-4-aminomethyl-4-(3,4*-dichlorophenyl)piperidine (260.8 mg, 0.726 mmol), 3-cyano-2-methoxy-1-naphthoic acid (164.6 mg, 0.724 mmol), HOBT hydrate (290 mg, 1.89 mmol), N-methylmorpholine (0.17 mL), and DCM (15 mL) was added 1-(3-(dimethylamino)propyl-3-ethylcarbodiimide hydrochloride (215.5 mg, 1.12 mmol). After 72h, the mixture was diluted with 30% hexane/EtOAc, washed successively with water (2X), 0.1 N aq. HCl (2X), sat. aq. NaHCO₃, dried, filtered, and concentrated. The residue was purified by chromatography (0-1%-MeOH/DCM) to give the title compound as a white, foamy solid. MS m/z 468.
b) 1-N-BOC-4-aminomethyl-4-(3,4-dichlorophenyl)piperidine
   A mixture containing 1-N-BOC-4-(3,4-dichlorophenyl)-4-cyanopiperidine (5.25 g, 14.78 mmol), Raney Ni catalyst (5g of 50% aq. slurry), EtOH (175 mL), and ammonium hydroxide (88 mL) was placed under a hydrogen atmosphere (50 psi) and agitated (Parr apparatus) for 18 h. The mixture was filtered through diatomaceous earth, concentrated, and purified by chromatography (0-5% MeOH/DCM) to give the title compound as an off-white solid. MS m/z 344 (M+1-CH₃). 1H NMR (CDCl₃) δ 7.44 (d, 1H), 7.38 (d, 1H), 7.15 (m, I H), 3.7 (br d, 2H), 3.07 (m, 2H), 2.76 (s, 2H), 2.08 (br d, 2H), 1.71 (m, 2H), 1.44 (s, 9H).
c) 1-N-BOC-4-(3,4-dichlorophenyl)-4-cyanopiperidine
   A solution containing bis(2-chloroethyl)-N-BOC amine (described in US Patent 5,661,163) (8.15 g, 33.67 mmol), 3,4-dichlorophenylacetonitrile (5.05 g, 27.17 mmol), and DMSO (50 mL) was stirred at RT and solid cesium carbonate (17.6 g, 54.02 mmol) was added (in portions) over 10 minutes. After 20 h, additional cesium carbonate (1.7 g,) was added, and the mixture stirred for an additional 72 h. The mixture was partitioned between water and EtOAc, the aq. layer was removed, and the organic layer washed successively with additional water, 0.1M aq. HCl (2X), sat. aq. NaHCO₃, and brine. The organic layer was dried, filtered, concentrated, and the residue triturated (3:1 hexane/ethyl acetate) to give the title compound as an off-white solid, m.p. 142-145 °C. MS m/z 255 . 1H NMR (CDCl₃) δ 7.55 (d, 1H), 7.49 (d, 1H), 7.32 (m, 1H), 4.3 (br d, 2H), 3.18 (br t, 2H), 2.07 (d, 2H), 1.89 (m, 2H), 1.48 (s, 9H).

### Example 4: 1-N-Methyl-4-(3,4-dichlorophenyl)-4-(3-(3-cyano-2-methoxynaphth-1-yl)-3-oxo-2-azaprop-1-yl)piperidine.

The title compound of the following structure 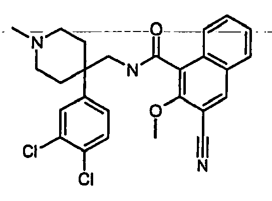 was prepared as a citrate, as follows. A solution containing 4-(3,4-dichlorophenyl)-4-(3-(3-cyano-2-methoxynaphth-1-yl)-3-oxo-2-azaprop-1-yl) piperidine (103 mg, 0.22 mmol), formic acid (0.25 mL), and 37% aq. formaldehyde (2 mL) was heated at 100°C. for 18h, then cooled and concentrated. The residue was partitioned between DCM and sat. aq. NaHCO₃ and the organic layer was removed. The basic aq. layer was extracted with additional DCM (2X), and the combined organic extracts were dried, filtered, and concentrated. The residue was purified by chromatography (Chromatotron - silica rotor) (5% MeOH/DCM w/0.5% aq. NH₃) and converted to the citrate salt to give the title compound as a white powder. MS m/z 482 (M+H).

### Example 5: 4-(4-Chlorophenyl)-4-(3-(3-cyano-2-methoxynaphth-1-yl)-3-oxo-2-azaprop-1-yl)piperidine.

The title compound of the following structure 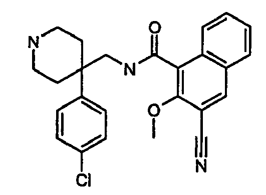 was prepared as a citrate, as follows. In the same manner as Example 3, but using 1-N-BOC-4-(4-chlorophenyl)-4-(3-(3-cyano-2-methoxynaphth-1-yl)-3-oxo-2-azaprop-1-yl)piperidine (350 mg, 0.655 mmol), the citrate salt was isolated by filtration from Et₂O to give the title compound as a white powder. MS m/z 434 (M+H).

The requisite 1-N-BOC-4-(4-chlorophenyl)-4-(3-(3-cyano-2-methoxynaphth-1-yl)-3-oxo-2-azaprop-1-yl)piperidine was prepared as follows:
a) 1-N-BOC-4-(4-chlorophenyl)-4-(3-(3-cyano-2-methoxynaphth-1-yl)-3-oxo-2-azaprop-1-yl)piperidine.
   In the same manner as Example 3a, but using 1-N-BOC-4-aminomethyl-4-(4-chlorophenyl) piperidine (244 mg, 0.75 mmol), 3-cyano-2-methoxy-1-naphthoic acid (170 mg, 0.748 mmol), HOBT hydrate (281 mg, 1.83 mmol), N-methylmorpholine (0.165 mL), 1-(3-(dimethylamino)propyl-3-ethylcarbodiimide hydrochloride (240 mg, 1.25 mmol), and DCM (10 mL), to yield the title compound as a foamy solid. MS m/z 434 .
b) 1-N-BOC-4-aminomethyl-4-(4-chlorophenyl) piperidine.
   In the same manner as Example 3b, but using 1-N-BOC-4-(4-chlorophenyl)-4-cyanopiperidine (1.05 g, 3.26 mmol), Raney Ni catalyst (1.4 g of 50% aq. slurry), EtOH (50 mL), and ammonium hydroxide (25 mL), to yield the title compound as a viscous oil. MS m/z 310 (M+H-Me).
c) 1-N-BOC-4-(4-chlorophenyl)-4-cyanopiperidine.
   A solution containing bis(2-chloroethyl)-N-BOC amine (3.72 g, 15.38 mmol), 4-chlorobenzyl cyanide (2.10 g, 13.88 mmol), and anhydrous DMF (15 mL) was stirred and NaH (60% dispersion in mineral oil) (1.6 g, 40 mmol) was added in portions over 1h. The mixture was heated at 60-65 °C. for 1h, stirred at RT for 72h, then was poured into ice/water and extracted with EtOAc (2X). The organic extracts were washed (water and brine), dried, filtered, and concentrated. The residue was purified by chromatography (8:1:1 hexane/DCM/EtOAc) to give the title compound as a yellow solid. MS m/z 221 .

### Example 6: 1-N-Methyl-4-(4-chlorophenyl)-4-(3-(3-cyano-2-methoxynaphth-1-yl)-3-oxo-2-azaprop-1-yl)piperidine.

The title compound of the following structure 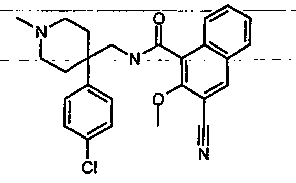 was prepared as a citrate, as follows. In the same manner as Example 4, but using 4-(4-chlorophenyl)-4-(3-(3-cyano-2-methoxynaphth-1-yl)-3-oxo-2-azaprop-1-yl)piperidine (71.5 mg, 0.165 mmol), the citrate salt was isolated by filtration from Et₂O to give the title compound as a white powder. MS m/z 448 (M+H).

### Example 7: 1-N-(2-Methoxyethyl)-4-(4-chlorophenyl)-4-(3-(3-cyano-2-methoxynaphth-1-yl)-3-oxo-2-azaprop-1-yl) piperidine.

The title compound of the following structure 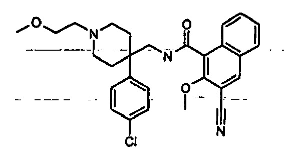 was prepared as a citrate, as follows. A solution containing 4-(4-chlorophenyl)-4-(3-(3-cyano-2-methoxynaphth-1-yl)-3-oxo-2-azaprop-1-yl) piperidine (38.5 mg, 0.089 mmol), 2-bromoethyl methyl ether (55.5 mg, 0.40 mmol), TEA (0.075 mL), and DMF (0.5 mL) was heated (microwave) at 60°C. for 1.25 h, stirred at RT overnight, diluted with EtOAc, then washed successively with water (2X) and sat. aq. NaHCO₃. The organic phase was dried, filtered, and concentrated. The residue was purified by chromatography (2-5% MeOH/DCM w/ 0.5% aq. NH₃), converted to the citrate salt, and isolated by filtration from Et₂O to give the title compound as a white powder. MS m/z 492 (M+H).

### Example 8: 4-(3,4-Dichlorophenyl)-4-(3-(3-cyano-2,4-dimethoxynaphth-1-yl)-3-oxo-2-azaprop-1-yl)piperidine.

The title compound of the following structure 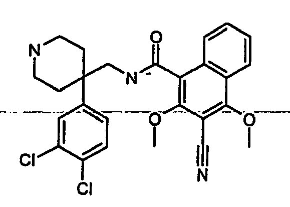 was prepared as a citrate, as follows. In the same manner as Example 3, but using 1-N-BOC-4-(3,4-dichlorophenyl)-4-(3-(3-cyano-2,4-dimethoxynaphth-1-yl)-3-oxo-2-azaprop-1-yl)piperidine (801 mg, 1.34 mmol), TFA (25 mL), and DCM (25 mL), the citrate salt of to yield the title compound as a white, foamy solid. MS m/z 498 (M+H).

The requisite 1-N-BOC-4-(3,4-dichlorophenyl)-4-(3-(3-cyano-2,4-dimethoxynaphth-1-yl)-3-oxo-2-azaprop-1-yl)piperidine was prepared as follows: 1-N-BOC-4-(3,4-dichlorophenyl)-4-(3-(3-cyano-2,4-dimethoxynaphth-1-yl)-3-oxo-2-azaprop-1-yl)piperidine.

A solution containing 3-cyano-2,4-dimethoxy-1-naphthoyl chloride (described in international publication WO 00/20389) (408.3 mg, 1.48 mmol) and dry DCM (2.5 mL) was added in portions (0.25 mL) to a stirred, cooled (ice bath) solution containing 1-N-BOC-4-(3,4-dichlorophenyl)-4-aminomethyl)piperidine (537 mg, 1.49 mmol), TEA (0.42 mL), and dry DCM (20 mL). After 1h, the reaction was warmed to RT, stirred an additional 1.5h, then concentrated. The residue was partitioned between water and EtOAc and the organic phase was removed and washed successively with 0.1N aq. HCl (2X), water, sat. aq. NaHCO₃ (2X), and brine. The organic phase was dried, filtered, concentrated, and the residue purified by chromatography (0-1% MeOH/DCM) to give the title compound as an off-white, foamy solid. MS m/z 498 .

### Example 9: 4-(3,4-Dichlorophenyl)-4-(3-(3-cyano-2-ethylnaphth-1-yl)-3-oxo-2-azaprop-1-yl)piperidine.

The title compound of the following structure 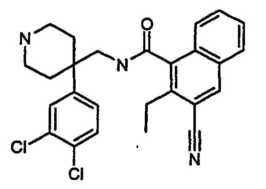 was prepared as a citrate, as follows. In the same manner as Example 3, but using 1-N-BOC-4-(3,4-dichlorophenyl)-4-(3-(3-cyano-2-ethylnaphth-1-yl)-3-oxo-2-azaprop-1-yl)piperidine (166.8 mg, 0.294 mmol), the citrate salt was isolated by filtration from Et₂O to give the title compound as a white powder. MS m/z 466 (M+H).

The requisite 1-N-BOC-4-(3,4-dichlorophenyl)-4-(3-(3-cyano-2-ethylnaphth-1-yl)-3-oxo-2-azaprop-1-yl)piperidine was prepared as follows:
1-N-BOC-4-(3,4-dichlorophenyl)-4-(3-(3-cyano-2-ethylnaphth-1-yl)-3-oxo-2-azaprop-1-yl)piperidine.

In the same manner as Example 3a, but using 1-N-BOC-4-aminomethyl-4-(3,4-dichlorophenyl) piperidine (375 mg, 1.04 mmol), 3-cyano-2-ethyl-1-naphthoic acid (described in international publication WO 00/20389, (233 mg, 1.04 mmol), HOBT hydrate (399 mg, 2.6 mmol), N-methylmorpholine (0.23 mL), 1-(3-(dimethylamino)propyl-3-ethylcarbodiimide hydrochloride (330 mg, 1.72 mmol), and DCM (10 mL), to yield the title compound as a foamy solid. MS m/z 466 .

### Example 10: 1-N-Methyl-4-(3,4-dichlorophenyl)-4-(3-(3-cyano-2-ethylnaphth-1-yl)-3-oxo-2 azaprop-1-yl)piperidine.

The title compound of the following structure 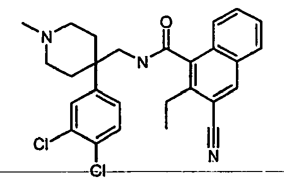 was prepared as a citrate, as follows. In the same manner as Example 4, but using 4-(3,4-dichlorophenyl)-4-(3-(3-cyano-2-ethylnaphth-1-yl)-3-oxo-2-azaprop-1-yl)piperidine (69 mg, 0.148 mmol), the citrate salt was isolated by filtration from Et₂O to give the title compound as a white powder. MS m/z 480 (M+H).

### Example 11: 1-N-Methyl-4-(4-fluorophenyl)-4-(3-(3-cyanonaphth-1-yl)-(3-oxo-2-azaprop-1-yl))piperidine.

The title compound of the following structure 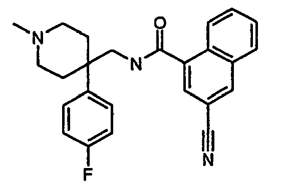 was prepared as a citrate salt as follows. To a solution containing 3-cyano-1-naphthoic acid (0.435 g, 2.21 mmol), 1-N-methyl-4-(4-fluorophenyl)-4-(aminomethyl)piperidine (0.539 g, 2.43 mmol), 1-ethyl-3-(3-dimethylaminopropyl)carbodiimide hydrochloride (0.676 g, 3.53 mmol) and 1-hydroxybenzotriazole (0.600 g, 4.44 mmol) in DCM (20 mL) was added TEA (0.92 mL, 6.60 mmol). The solution was stirred at room temperature overnight. The mixture was partitioned between DCM and sat. NaHCO₃, the organic layer was removed, and the aq. layer extracted with DCM (2x). The organic extracts were combined, dried, filtered, and concentrated. The residue was purified by chromatography (1-5% MeOH-DCM w/1% aq. NH₃) to give the title compound as a white solid (0.7 g, 79% yield). MS m/z 402.50 (M+H). The citrate salt was obtained by standard procedure.

The requisite 1-N-methyl-4-(4-fluorophenyl)-4-(aminomethyl)piperidine was prepared as follows:
a) 1-N-Methyl-4-(4-fluorophenyl)-4-cyanopiperidine
   To a solution containing mechlorethamine hydrochloride (1.923 g, 9.99 mmol) and 4-fluorophenyl acetonitrile (1.35 g, 9.99 mmol) in DMF (30 mL) was added sodium hydride (1.6 g, 40 mmol) slowly at 0 °C. The resulting suspension was stirred and heated at 60°C for 24 hrs. The reaction mixture was quenched with ice water, extracted with EtOAc (3x). The organic extracts were combined, washed with sat. NaCl (3x), dried, filtered, and concentrated. The residue was purified by chromatography (2-5% MeOH-DCM) to give the title compound as a yellow oil (1.788 g, 82% yield). MS m/z 219.38 (M+H).
b) 1-N-Methyl-4-(4-fluorophenyl)-4-(aminomethyl)piperidine
   To a solution of 1-N-methyl-4-(4-fluorophenyl)-4-cyanopiperidine (1.788 g, 8.20 mmol) in dry THF (25 mL) was added LAH (1M in THF, 25mL, 24.6 mmol). The solution was stirred at room temperature overnight. The reaction was quenched by adding water (2.5 mL), followed by 15% NaOH (2.5 mL) and water (2.5 mL). The mixture was then filtered through diatomaceous earth, washed with EtOAc, dried, filtered, and concentrated to give the title compound as a yellow oil (1.619 g, 89% yield). MS m/z 223.45 (M+H).

### Example-12: 1-N-Methyl-4-(4-fluorophenyl)-4-(3-(3-cyano-2-methoxynaphth-1-yl)-(3-oxo-2-azaprop-1-yl))piperidine.

The title compound of the following structure 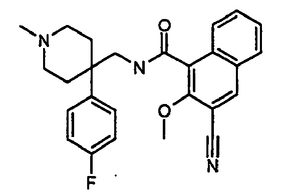 was prepared as a citrate salt in the same manner as Example 11, but using 3-cyano-2-methoxy-1-naphthoic acid (100 mg, 0.44 mmol), 1-N-methyl-4-(4-fluorophenyl)-4-(aminomethyl)piperidine (107 mg, 0.48 mmol), 1-ethyl-3-(3-dimethylaminopropyl)carbodiimide hydrochloride (135 mg, 0.704 mmol), 1-hydroxybenzotriazole (119 mg, 0.88 mmol), DCM (5 mL), and TEA (0.184 mL, 1.32 mmol), to yield the title compound as a white solid. 74% yield, MS m/z 432.46 (M+H).

### Example 13: 1-N-Methyl-4-(4-fluorophenyl)-4-(3-(3-cyano-2-ethylnaphth-1-yl)-(3-oxo-2-azaprop-1-yl))piperidine.

The title compound of the following structure 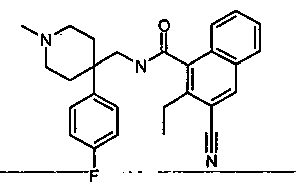 was prepared as a citrate salt as follows. To a solution containing 1-N-methyl-4-(4-fluorophenyl)-4-(aminomethyl)piperidine (98 mg, 0.441 mmol) and TEA (0.13 mL, 0.933 mmol) in DCM (5 mL) was added 3-cyano-2-ethyl-1-naphthoyl chloride (108 mg, 0.443 mmol) in DCM (1 mL) at 0 °C. The solution was stirred at 0 °C for 30 min and room temperature overnight. The mixture was partitioned between DCM and sat. NaHCO₃, the organic layer was removed, and the aq. layer extracted with DCM (2x). The organic extracts were combined, dried, filtered, and concentrated. The residue was purified by chromatography (1-5% MeOH-DCM w/1% aq. NH₃) to give the title compound as a light yellow solid (156 mg, 82% yield). MS m/z 430.51 (M+H). The citrate salt was obtained by standard procedure.

### Example 14: 1-N-Methyl-4-(-4-fluorophenyl)-4-(3-(3-cyanonaphth-1-yl)-(3-oxo-2-N-methyl-2-azaprop-1-yl))piperidine.

The title compound of the following structure 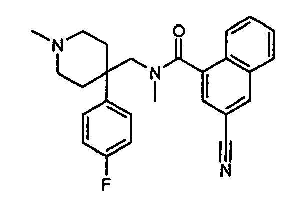 was prepared as a citrate salt as follows. To a solution of 1-N-methyl-4-(4-fluorophenyl)-4-(3-(3-cyanonaphth-1-yl)-(3-oxo-2-azaprop-1-yl))piperidine (366 mg, 0.912 mmol) in dry DMF (9 mL) was added NaH (44 mg, 1.1 mmol). The mixture was stirred at room temperature for 30 min and cooled to 0 °C. Methyl iodide (0.085 mL, 1.36 mmol) was added and the mixture was stirred at 0 °C for 30 min, room temperature overnight. The mixture was partitioned between EtOAc and water, the organic layer was removed, and the aq. layer extracted with EtOAc (2x). The organic extracts were combined, washed with sat. NaCl (3x), dried, filtered, and concentrated. The residue was purified by chromatography (1-5% MeOH-DCM w/1% aq. NH₃) to give the title compound as a white solid (164 mg, 43% yield). MS m/z 416.54 (M+H). The citrate salt was obtained by standard procedure.

### Examples 15 - 38:

The compounds of Examples 15 through 38 were prepared by processes similar to those given in Examples 11-14 but with replacement of 4-fluorophenyl acetonitrile with an appropriately substituted phenyl acetonitrile, compounds of Examples 15 through 38 and intermediates listed in Table 2 were obtained.

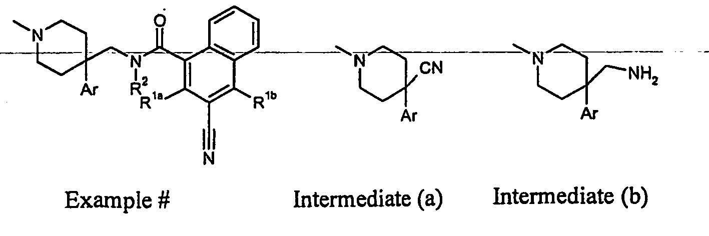

**Table 2**

| **Example #** | **Ar** | **R^{1a}** | **R²** | **R^{1b}** | **Yield (%)** | **MS m/z (M+H)** |
|---|---|---|---|---|---|---|
| 11 | 4-fluorophenyl | H | H | H | 79 | 402.50 |
| 11 (a) | 4-fluorophenyl | | | | 82 | 219.38 |
| 11 (b) | 4-fluorophenyl | | | | 89 | 223.45 |
| 12 | 4-fluorophenyl | OMe | H | H | 74 | 432.46 |
| 13 | 4-fluorophenyl | Et | H | H | 82 | 430.51 |
| 14 | 4-fluorophenyl | H | Me | H | 43 | 416.54 |
| 15 | 3,4-difluorophenyl | H | H | H | 81 | 420.52 |
| 15 (a) | 3,4-difluorophenyl | | | | 77 | 237.41 |
| 15 (b) | 3,4-difluorophenyl | | | | 92 | 241.45 |
| 16 | 3,4-difluorophenyl | OMe | H | H | 74 | 450.46 |
| 17 | 3,4-difluorophenyl | Et | H | H | 44 | 448.51 |
| 18 | 3,4-difluorophenyl | H | Me | H | 50 | 434.44 |
| 19 | 4-methoxyphenyl | H | H | H | 78 | 414.53 |
| 19 (a) | 4-methoxyphenyl | | | | 100 | 231.46 |
| 19 (b) | 4-methoxyphenyl | | | | 93 | 235.49 |
| 20 | 4-methoxyphenyl | OMe | H | H | 77 | 444.50 |
| 21 | 4-methoxyphenyl | Et | H | H | 31 | 442.54 |
| 22 | 4-methoxyphenyl | H | Me | H | 37 | 428.54 |
| 23 | 3,4-dimethoxyphenyl | H | H | H | 67 | 444.52 |
| 23 (a) | 3,4-dimethoxyphenyl | | | | 92 | 261.49 |
| 23 (b) | 3,4-dimethoxyphenyl | | | | 86 | 265.52 |
| 24 | 3,4-dimethoxyphenyl | OMe | H | H | 76 | 474.49 |
| 25 | 3,4-dimethoxyphenyl | Et | H | H | 23 | 472.54 |
| 26 | 3,4-dimethoxyphenyl | H | Me | H | 47 | 458.53 |
| 27 | 3,4-methylenedioxyphenyl | H | H | H | 83 | 428.51 |
| 27 (a) | 3,4-methylenedioxyphenyl | | | | 88 | 245.44 |
| 27 (b) | 3,4-methylenedioxyphenyl | | | | 90 | 249.46 |
| 28 | 3,4-methylenedioxyphenyl | OMe | H | H | 83 | 458.48 |
| 29 | 3,4-methylenedioxyphenyl | Et | H | H | 62 | 456.40 |
| 30 | 3,4-methylenedioxyphenyl | H | Me | H | 21 | 442.40 |
| 31 | 4-difluoromethoxyphenyl | H | H | H | 74 | 450.53 |
| 31 (a) | 4-difluoromethoxyphenyl | | | | 81 | 267.43 |
| 31 (b) | 4-difluoromethoxyphenyl | | | | 62 | 271.48 |
| 32 | 4-difluoromethoxyphenyl | OMe | H | H | 72 | 480.51 |
| 33 | 4-difluoromethoxyphenyl | Et | H | H | 39 | 478.54 |
| 34 | 4-difluoromethoxyphenyl | H | Me | H | 38 | 464.48 |
| 35 | 4-trifluoromethylphenyl | H | H | H | 75 | 452 |
| 35 (a) | 4-trifluoromethylphenyl | | | | 79 | 269 |
| 35 (b) | 4-trifluoromethylphenyl | | | | 78 | 273.5 |
| 36 | 4-trifluoromethylphenyl | OMe | H | H | 73 | 482 |
| 37 | 4-trifluoromethylphenyl | OMe | H | OMe | 73 | 512 |
| 38 | 4-trifluoromethylphenyl | Et | H | H | 73 | 480 |

### Example 39: 4-(4-Fluorophenyl)-4-(3-(3-cyanonaphth-1-yl)-3-oxo-2-azaprop-1-yl)piperidine.

The title compound of the following structure 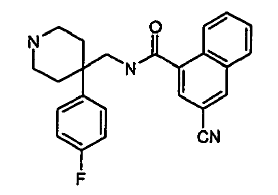 was prepared as a citrate in the same manner as Example 3, but using 1-N-BOC-4-(4-fluorophenyl)-4-(3-(3-cyanonaphth-1-yl)-3-oxo-2-azaprop-1-yl)piperidine (158 mg, 0.324 mmol), to yield the title compound as a white powder. MS m/z 388 (M+H).

The requisite 1-N-BOC-4-(4-fluorophenyl)-4-(3-(3-cyanonaphth-1-yl)-3-oxo-2-azaprop-1-yl)piperidine was prepared as follows:
a) 1-N-BOC-4-(4-fluorophenyl)-4-(3-(3-cyanonaphth-1-yl)-3-oxo-2-azaprop-1-yl)piperidine.
   In the same manner as Example 3a, but using 1-N-BOC-4-amionmethyl-4-(4-fluoro)piperidine (1.68 g, 5.45 mmol), 3-cyano-1-naphthoic acid (980 mg, 4.97 mmol), HOBT (1.34 g, 9.92 mmol), triethylamine (2.08 mL), 1-(3-(dimethylamino)propyl-3-ethylcarbodiimide hydrochloride (1.52 g, 7.93 mmol), and DCM (30 mL), to yield the title compound as a foamy solid. MS m/z 388 (M+H-BOC).
b)1-N-BOC-4-aminomethyl-4-(4-fluorophenyl) piperidine.
   In the same manner as Example 3b, but using 1-N-BOC-4-(4-fluorophenyl)-4-cyanopiperidine (4.64 g, 15.2 mmol), Raney Ni catalyst (1.4 g of 50% aq. slurry), EtOH (30 mL), and ammonium hydroxide (20 mL), to yield the title compound as a viscous oil. MS m/z 209 (M+H-BOC).
c) I -N-BOC-4-(4-fluorophenyl)-4-cyanopiperidine.
   To a solution of 4-(4-fluorophenyl)-4-cyanopiperidine (3.63 g, 17.8 mmol) in THF (90 mL) was added BOC-anhydride (3.88 g, 17.8 mmol) and DIPEA (3.10 mL, 17.8 mmol). The resulting solution was stirred at room temperature for overnight and poured into 0.1 N HCl. The aq. layer was extracted with EtOAc (2x80 mL). Combined EtOAc were dried over MgSO₄, filtered and concentrated. The residue was purified by chromatography (1% MeOH/DCM) to give the title compound as a yellow oil. MS m/z 205 (M+H-BOC).
d) 4-(4-fluorophenyl)-4-cyanopiperidine
   A solution containing bis(2-chloroethyl)amine hydrochloride (4.0 g, 22.4 mmol), 4-fluorobenzyl cyanide (3.03 g, 22.4 mmol), and anhydrous DMF (100 mL) was stirred at 0 °C and NaH (60% dispersion in mineral oil) (3.6 g, 90 mmol) was added in portions. The mixture was heated at 60°C overnight, then was poured into ice/water and extracted with EtOAc (2X). The organic extracts were washed (water and brine), dried, filtered, and concentrated. The residue was purified by chromatography (1% MeOH/DCM) to give the title compound as a yellow oil. MS m/z 205 (M+H).

### Example 40: 4-(4-Fluorophenyl)-4-(3-(3-cyanonaphth-1-yl)-(3-oxo-2-N-methyl-2-azaprop-1-yl))piperidine.

The title compound of the following structure 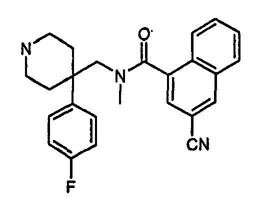 was prepared as a citrate in the same manner as Example 3, but using 1-N-BOC-4-(4-fluorophenyl)-4-(3-(3-cyanonaphth-1-yl)-(3-oxo-2-N-methyl-2-azaprop-1-yl))piperidine (1.5 g, 2.99 mmol), to yield the title compound as a white powder. MS m/z 402 (M+H).

The requisite 1-N-BOC-4-(4-fluorophenyl)-4-(3-(3-cyanonaphth-1-yl)-3-oxo-2-N-methyl-2-azaprop-1-yl))piperidine was prepared in the same manner as Example 14 but using 1-N-BOC-4-(4-fluorophenyl)-4-(3-(3-cyanonaphth-1-yl)-3-oxo-2-azaprop-1-yl)piperidine instead of 1-N-methyl-4-(4-fluorophenyl)-4-(3-(3-cyanonaphth-1-yl)-3-oxo-2-azaprop-1-yl)piperidine.

### Example 41: 4-(4-Fluorophenyl)-4-(3-(3-cyano-2-methoxynaphth-1-yl)-(3-oxo-2-azaprop-1-yl))piperidine.

The title compound of the following structure 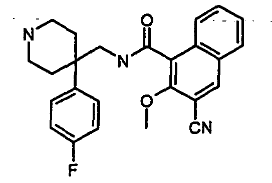 was prepared as a citrate in the same manner as Example 3, but using 3-cyano-2-methoxy-1-naphthoic acid instead of 3-cyano-1-naphthoic acid in 3a, to yield the title compound as a white powder. MS m/z 418 (M+H).

### Example 42: 4-(4-Fluorophenyl)-4-(3-(3-cyano-2-ethylnaphth-1-yl)-(3-oxo-2-azaprop-1-yl))piperidine.

The title compound of the following structure 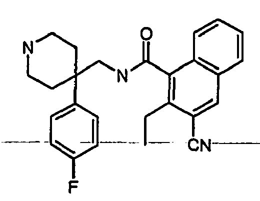 was prepared as a citrate in the same manner as Example 3, to yield the title compound as a white powder. MS m/z 416 (M+H).

The requisite 1-N-BOC-4-(4-fluorophenyl)-4-(3-(3-cyano-2-ethylnaphth-1-yl)-3-oxo-2-azaprop-1-yl)piperidine was prepared in the same manner as Example 13, but using 1-N-BOC-4-amionmethyl-4-(4-fluoro)piperidine instead of 1-N-methyl-4-amionmethyl-4-(4-fluoro)piperidine.

### Example 43: 4-(4-Fluorophenyl)-4-(3-(3-cyano-2,4-dimethoxynaphth-1-yl)-(3-oxo-2-azaprop-1-yl))piperidine.

The title compound of the following structure 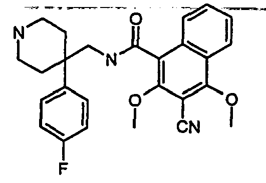 was prepared as a citrate in the same manner as Example 42, but using 3-cyano-2,4-dimethoxy-1-naphthoyl chloride instead of 3-cyano-2-ethyl-1-naphthoyl chloride, to yield the title compound as a white powder. MS m/z 448 (M+H).

### Example 44: 1-N-Methyl-4-(4-fluorophenyl)-4-(3-(3-cyano-2,4-dimethoxynaphth-1-yl)-(3-oxo-2-azaprop-1-yl))piperidine.

The title compound of the following structure 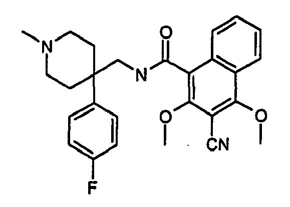 was prepared as a citrate in the same manner as Example 13, but using 3-cyano-2,4-dimethoxy-1-naphthoyl chloride instead of 3-cyano-2-ethyl-1-naphthoyl chloride, to yield the title compound as a white powder. MS m/z 462 (M+H).

### Example 45: 1-N-Methyl-4-(4-fluorophenyl)-4-(3-(4-fluoronaphth-1-yl)-(3-oxo-2-azaprop-1-yl))piperidine.

The title compound of the following structure 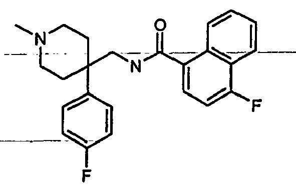 was prepared as a citrate in the same manner as Example 11, but using 4-fluoro-1-naphthoic acid instead of 3-cyano-1-naphthoic acid, to yield the title compound as a white powder. MS m/z 395 (M+H).

### Example 46: 1-N-Methyl-4-(4-fluorophenyl)-4-(3-(4-fluoronaphth-1-yl)-(3-oxo-2-N-methyl-2-azaprop-1-yl))piperidine.

The title compound of the following structure 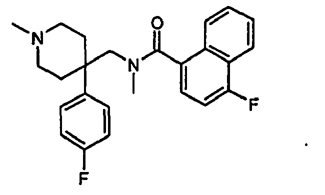 was prepared as a citrate in the same manner as Example 14, but using 1-N-methyl-4-(4-fluorophenyl)-4-(3-(4-fluoronaphth-1-yl)-(3-oxo-2-azaprop-1-yl))piperidine instead of 1-N-methyl-4-(4-fluorophenyl)4-(3-(3-cyanonaphth-1-yl)-(3-oxo-2-azaprop-1-yl))piperidine, to yield the title compound as a white powder. MS m/z 409 (M+H).

### Example 47: 1-N-Methyl-4-(3,4-difluorophenyl)-4-(3-(3-cyano-2,4-dimethoxynaphth-1-yl)-(3-oxo-2-azaprop-1-yl))piperidine.

The title compound of the following structure 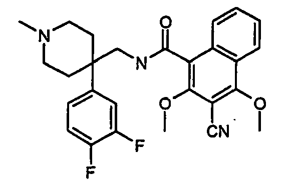 was prepared as a citrate in the same manner as Example 45, but using 1-N-methyl-4-(3,4-difluorophenyl)-4-(aminomethyl)piperidine instead of 1-N-methyl-4-(4-fluorophenyl)-4--(aminomethyl)piperidine, to yield the title compound as a light yellow powder. MS m/z 480 (M+H).

### Example 46: 1-N-Ethyl-4-(4-fluorophenyl)-4-(3-(3-cyanonaphth-1-yl)-3-oxo-2-azaprop-1-yl)piperidine.

The title compound of the following structure 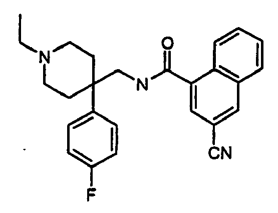 was prepared as a citrate in the same manner as Example 11, but using 1-N-ethyl-4-(4-fluorophenyl)-4-(aminomethyl)piperidine instead of 1-N-methyl-4-(4-fluorophenyl)-4-(aminomethyl)piperidine, to yield the title compound as a white powder. MS m/z 416 (M+H).

The requisite 1-N-ethyl-4-(4-fluorophenyl)-4-(aminomethyl)piperidine was prepared as follows:
a) 1-N-Ethyl-4-(4-fluorophenyl)-4-(aminomethyl)piperidine
   To a solution of 1-N-(1-oxoethyl)-4-(4-fluorophenyl)-4-cyanopiperidine (3.4 g, 13.8 mmol) in THF (50 mL) was added LAH (1 N in THF, 55 mL, 55.2 mmol). The solution was heated to 70 °C for 3 hours. The reaction was quenched by adding water (5.5 mL), 15% NaOH (5.5 mL) and water (5.5 mL). The mixture was filtered through diatomaceous earth, washed with EtOAc. The organic layer was dried, filtered and concentrated. The residue was purified by chromatography (5%, 10% MeOH/DCM, 20% MeOH/DCM with 2% NH₄OH) to give the title compound as a yellow oil. MS m/z 237 (M+H).
b) 1-N-(1-Oxoethyl)- 4-(4-fluorophenyl)-4-cyanopiperidine
   To a solution of 4-(4-fluorophenyl)-4-cyanopiperidine (3.33 g, 16.3 mmol) and triethylamine (4.77 mL, 34.2 mmol) in DCM (90 mL) was added acetyl chloride (1.16 mL, 16.3 mmol) at 0 °C The solution was stirred at 0 °C for 0.5 hour and room temperature for 17 h. NaHCO₃ (sat.) was added. The mixture was extracted with DCM (2x), dried, filtered and concentrated. The residue was purified by chromatography (30%, 50%, 60%, 80% and 90% EtOAc/hexane) to give the title compound as a yellow oil. MS m/z 247 (M+H).

### Example 49: 1-N-Ethyl-4-(4-fluorophenyl)-4-(3-(3-cyano-2-methoxynaphth-1-yl)-3-oxo-2-azaprop-1-yl)piperidine.

The title compound of the following structure 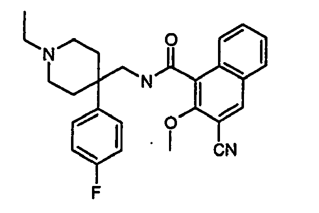 was prepared as a citrate in the same manner as Example 12, but using 1-N-ethyl-4-(4-fluorophenyl)-4-(aminomethyl)piperidine instead of 1-N-methyl-4-(4-fluorophenyl)-4-(aminomethyl)piperidine, to yield the title compound as a white powder. MS m/z 446 (M+H).

### Example 50: 1-N-Ethyl-4-(4-fluorophenyl)-4-(3-(3-cyanonaphth-1-yl)-3-oxo-2-N-methyl-2-azaprop-1-yl)piperidine.

The title compound of the following structure 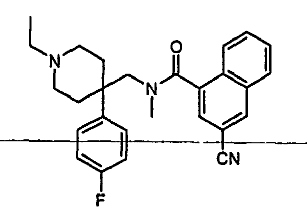 was prepared as a citrate in the same manner as Example 14, but using 1-N-ethyl-4-(4-fluorophenyl)-4-(3-(3-cyanonaphth-1-yl)-(3-oxo-2-azaprop-1-yl))piperidine instead of 1-N-methyl-4-(4-fluorophenyl)-4-(3-(3-cyanonaphth-1-yl)-(3-oxo-2-azaprop-1-yl))piperidine, to yield the title compound as a white powder. MS m/z 430 (M+H).

### Example 51: 1-N-Ethyl-4-(4-fluorophenyl)-4-(3-(3-cyano-2-ethylnaphth-1-yl)-3-oxo-2-azaprop-1-yl)piperidine.

The title compound of the following structure 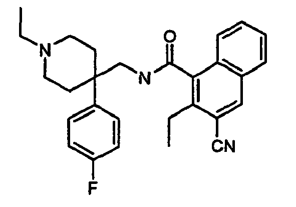 was prepared as a citrate in the same manner as Example 13, but using 1-N-ethyl-4-(4-fluorophenyl)-4-(aminomethyl)piperidine instead of 1-N-methyl-4-(4-fluorophenyl)-4-(aminomethyl)piperidine, to yield the title compound as a light yellow powder. MS m/z 444 (M+H).

### Example 52: 1-N-Methyl-4-(4-fluorophenyl)-4-(3-(3-cyano-2,4-dimethoxynaphth-1-yl)-(3-oxo-2-azaprop-1-yl))piperidine.

The title compound of the following structure 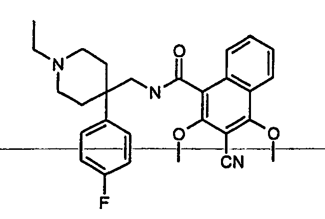 was prepared as a citrate in the same manner as Example 45, but using 1-N-ethyl-4-(4-fluorophenyl)-4-(aminomethyl)piperidine instead of 1-N-methyl-4-(4-fluorophenyl)-4-(aminomethyl)piperidine, to yield the title compound as a white powder. MS m/z 476 (M+H).

### Example 53: 1-N-Ethyl-4-(3,4-dichlorophenyl)-4-(3-(3-cyano-2-methoxynaphth-1-yl)-3-oxo-2-azaprop-1-yl)piperidine.

The title compound of the following structure 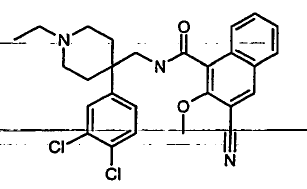 was prepared as a citrate salt, as follows. In the same manner as Example 13, but using 1-N-ethyl-4-aminomethyl-4-(3,4-dichlorophenyl)piperidine (76 mg, 0.265 mmol) and 3-cyano-2-methoxy-1-naphthoyl chloride (71 mg, 0.29 mmol), the citrate salt was isolated by filtration from Et₂O to give the title compound (116 mg) (63%) as a white powder. MS m/z 496 (M+H).

The requisite 1-N-ethyl-4-aminomethyl-4-(3,4-dichlorophenyl)piperidine was prepared as follows:
a) 1-N-ethyl-4-aminomethyl-4-(3,4-dichlorophenyl)piperidine
   To a stirred solution containing 1-N-(1-oxoethyl)- 4-(3,4-dichlorophenyl)-4-cyanopiperidine (623 mg, 2.097 mmol) and dry THF (10 mL), a solution of BH₃ in THF (20 mL of 1M) was slowly added. The solution was heated under reflux for 21 h, cooled to ambient, and cautiously treated with 1 N aq. HCl (10 mL). After 0.5 h, the volume was reduced by evaporation, sat. aq. NaHCO₃ was added (until basic), and the mixture was extracted with DCM (4X). The extracts were dried (MgSO₄), filtered, and concentrated. The residue was treated with 8 N aq. HCl (25 mL), stirred with intermittent heating for 72 h., basified (33% aq. NaOH), and extracted with DCM (4X). The DCM extracts were washed (brine), dried (MgSO₄), filtered, and concentrated. The residue was purified by chromatography (0-5% MeOH/DCM w/ 1%NH₃) to give the title compound (315 mg) (52%) as an off-white solid. MS m/z 287 (M+H).
b) 1-N-(1-oxoethyl)-4-(3,4-dichlorophenyl)-4-cyanopiperidine
   A stirred solution containing 4-(3,4-dichlorophenyl)-4-cyanopiperidine (535 mg, 2.097 mmol), TEA (0.44 mL), and dry DCM (15 mL) was cooled (ice bath) and acetyl chloride (210 mg, 2.67 mmol) was added dropwise. After 1h, the mixture was warmed to RT, stirred for an additional 18 h, diluted with DCM, and washed with sat. aq. NaHCO₃. The organic phase was dried (MgSO₄), filtered, and concentrated. The residue was purified by chromatography (0-2% MeOH/DCM) to give the title compound (quantitative) as an off-white, solid. MS m/z 297 (M+H).
c) 4-(3,4-dichlorophenyl)-4-cyanopiperidine
   A solution containing 1-N-BOC-4-(3,4-dichlorophenyl)-4-cyanopiperidine (Example 3c) (5.54 g, 15.59 mmol), TFA (50 mL), and DCM (50 mL) was stirred at RT for 18 h, then concentrated. The residue was treated with water, sodium bicarbonate, and sat. aq. sodium bicarbonate (until basic), then extracted with DCM (3X). The DCM extracts were dried (Na₂SO₄), filtered, and concentrated. The residue was purified by chromatography (0-5% MeOH/DCM) to give the title compound (3.81g) (95%) as an off-white solid. MS m/z 255 (M+H).

### Example 54: 1-N-Ethyl-4-(3,4-dichlororphenyl)-4-(3-(3-cyanonaphth-1-yl)-3-oxo-2-azaprop-1-yl)piperidine.

The title compound of the following structure 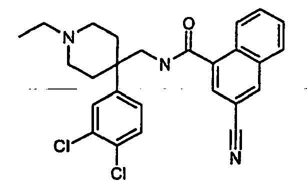 was prepared as a citrate salt, as follows. In the same manner as Example 13, but using 1-N-ethyl-4-aminomethyl-4-(3,4-dichlorophenyl)piperidine (151 mg, 0.525 mmol) and 3-cyano-1-naphthoyl chloride (124 mg, 0.577 mmol), the citrate salt was isolated by filtration from Et₂O to give the title compound 115 mg) (76%) as a white powder. MS m/z 466 (M+H).

### Example 55: 1-N-Methul-4-(3,4-dichlorophenyl)-4-(3-(3-cyanonaphth-1-yl)-3-oxo-2-azaprop-1-yl)piperidine.

The title compound of the following structure 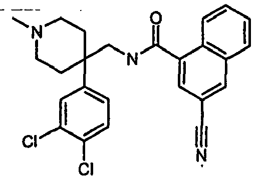 was prepared as a citrate salt, as follows. In the same manner as Example 11, but using 1-N-methyl-4-aminomethyl-4-(3,4-dichlorophenyl)piperidine (237 mg, 0.867 mmol) and 3-cyano-1-naphthoic acid (168 mg, 0.852 mmol), the citrate salt was isolated by filtration from Et₂O to give the title compound (306 mg) (57%) as a white powder. MS m/z 452 (M+H).

### Example 56: 1-N-Methyl-4-(3-fluorophenyl)-4-(3-(3-cyanonaphth-1-yl)-3-oxo-2-azaprop-1-yl)piperidine.

The title compound of the following structure 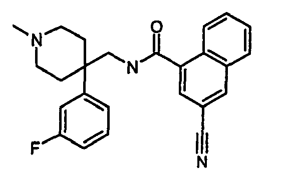 was prepared as follows. In the same manner as Example 13, but using 1-N-methyl-4-aminomethyl-4-(3-fluorophenyl)piperidine (172 mg, 0.775 mmol) and 3-cyano-1-naphthoyl chloride (164.5 mg, 0.763 mmol), the title compound (139 mg) (45%) was obtained as a white powder. MS m/z 402 (M+H).

The requisite 1-N-methyl-4-aminomethyl-4-(3-fluorophenyl)piperidine was prepared as follows:
a) 1-N-methyl-4-cyano-4-(3-fluorophenyl)piperidine
   In the same manner as Example 11a, but using 3-fluorophenylacetonitrile (5.05 g, 37.4 mmol), and following short-path distillation, the title compound (7.96 g) (97%) was obtained as a colorless liquid. MS m/z 219 (M+H).
b) 1-N-methyl-4-aminomethyl-4-(3-fluorophenyl)piperidine
   In the same manner as Example 11b, but using 1-N-methyl-4-cyano-4-(3--fluorophenyl)piperidine (3.08 g, 14.1 mmol), and following short-path distillation, the title compound (2.95 g) (94%) was obtained as a colorless liquid. MS m/z 223 (M+H).

### Example 57: 1-N-Methyl-4-(3-fluorophenyl)-4-(3-(3-cyano-2-methoxynaphth-1-yl)-3-oxo-2-azaprop-1-yl-)piperidine.

The title compound of the following structure 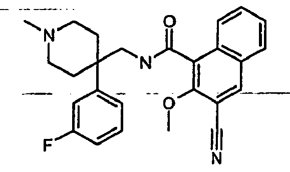 was prepared as a citrate salt, as follows. In the same manner as Example 13, but using 1-N-methyl-4-aminomethyl-4-(3-fluorophenyl)piperidine (166.2 mg, 0.748 mmol) and 3-cyano-2-methoxy-1-naphthoyl chloride (178.8 mg, 0.728 mmol), the citrate salt was isolated by filtration from Et₂O to give the title compound (325 mg) (72%) as a white powder. MS m/z 432 (M+H).

### Example 58: 1-N-Methyl-4-(3-fluorophenyl)-4-(3-(3-cyano-2-ethylnaphth-1-yl)-3-oxo-2-azaprop-1-yl)piperidine.

The title compound of the following structure 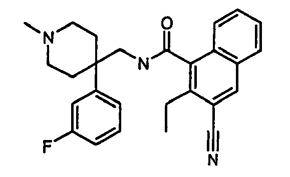 was prepared as a citrate salt, as follows. In the same manner as Example 13, but using 1-N-methyl-4-aminomethyl-4-(3-fluorophenyl)piperidine (151.9 mg, 0.683 mmol) and 3-cyano-2-ethyl-1-naphthoyl chloride (163.2 mg, 0.67 mmol), the citrate salt was isolated by filtration from Et₂O to give the title compound (272 mg) (65%) as a white powder. MS m/z 430 (M+H).

### Example 59: 1-N-Methyl-4-(3-fluorophenyl)-4-(3-(3-cyano-2,4-dimethoxynaphth-1-yl)-3-oxo-2-azaprop-1-yl)piperidine.

The title compound of the following structure 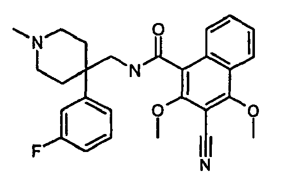 was prepared as a citrate salt, as follows. In the same manner as Example 13, but using 1-N-methyl-4-aminomethyl-4-(3-fluorophenyl)piperidine (154.8 mg, 0.696 mmol) and 3-cyano-2,4-dimethoxy-1-naphthoyl chloride (187.3 mg, 0.679 mmol), the citrate salt was isolated by filtration from Et₂O to give the title compound (247 mg) (55%) as a white powder. MS m/z 462 (M+H).

### Example 60: 1-N-Methyl-4-phenyl-4-(3-(3-cyanonaphth-1-yl)-3-oxo-2-azaprop-1-yl)piperidine.

The title compound of the following structure 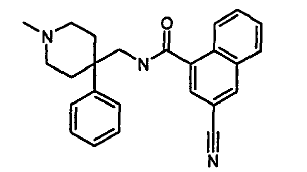 was prepared as a citrate salt, as follows. In the same manner as Example 13, but using 1-N-methyl-4-aminomethyl-4-phenylpiperidine (159 mg, 0.776 mmol) and 3-cyano-1-naphthoyl chloride (164.5 mg, 0.763 mmol), the citrate salt was isolated by filtration from Et₂O to give the title compound (278 mg) (65%) as a white powder. MS m/z 384 (M+H).

The requisite 1-N-methyl-4-aminomethyl-4-phenylpiperidine was prepared as follows:
a) 1-N-methyl-4-cyano-4-phenylpiperidine
   In the same manner as Example 11a, but using phenylacetonitrile (4.4 g, 37.6 mmol), and following short-path distillation, the title compound (7.05 g) (93%) was isolated as a colorless liquid. MS m/z 201 (M+H).
b) 1-N-methyl-4-aminomethyl-4-phenylpiperidine
   In the same manner as Example 1c, but using 1-N-methyl-4-cyano-4-phenylpiperidine (3.09 g, 15.4 mmol) and following short-path distillation, the title compound (2.71 g) (94%) was isolated as a colorless-liquid. MS m/z 205 (M+H).

### Example 61: 1-N-Methyl-4-phenyl-4-(3-(3-cyano-2-methoxynaphth-1-yl)-3-oxo-2-azaprop-1-yl)piperidine.

The title compound of the following structure 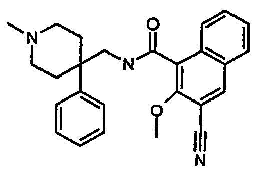 was prepared as a citrate salt, as follows. In the same manner as Example 13, but using 1-N-methyl-4-aminomethyl-4-phenylpiperidine (151 mg, 0.738 mmol) and 3-cyano-2-methoxy-1-naphthoyl chloride (175 mg, 0.713 mmol), the citrate salt was isolated by filtration from Et₂O to give the title compound (381 mg) (90%) as a white powder. MS m/z 414 (M+H).

### Example 62: 1-N-Methyl-4-phenyl-4-(3-(3-cyano-2-ethylnaphth-1-yl)-3-oxo-2-azaprop-1-yl)piperidine.

The title compound of the following structure 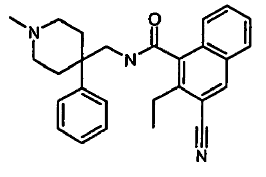 was prepared as a citrate salt, as follows. In the same manner as Example 13, but using 1-N-methyl-4-aminomethyl-4-phenylpiperidine (140.4 mg, 0.687 mmol) and 3-cyano-2-ethyl-1-naphthoyl chloride (163.2 mg, 0.67 mmol), the citrate salt was isolated by filtration from Et₂O to give the title compound (257 mg) (64%) as a white powder. MS m/z 412 (M+H).

### Example 63: 1-N-Methyl-4-phenyl-4-(3-(3-cyano-2,4-dimethoxynaphth-1-yl)-3-oxo-2-azaprop-1-yl)piperidine.

The title compound of the following structure 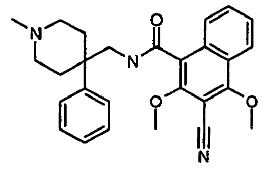 was prepared as a citrate salt, as follows. In the same manner as Example 13, but using 1-N-methyl-4-aminomethyl-4-phenylpiperidine (147.5 mg, 0.722 mmol) and 3-cyano-2,4-dimethoxy-1-naphthoyl chloride (187.3 mg, 0.679 mmol), the citrate salt was isolated by filtration from Et₂O to give the title compound (171 mg) (39%) as a white powder. MS m/z 444 (M+H).

### Example 64: 1-N-Methyl-4-phenyl-4-(3-(3-cyanonaphth-1-yl)-(3-oxo-2-N-methyl-2-azaprop-1-yl))piperidine.

The title compound of the following structure 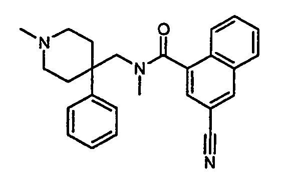 was prepared as a citrate salt, as follows. In the same manner as Example 13, but using 1-N-methyl-4-(N-methylaminomethyl)-4-phenylpiperidine (109 mg, 0.497 mmol) and 3-cyano-1-naphthoyl chloride (107 mg, 0.497 mmol), the citrate salt was isolated by filtration from Et₂O to give the title compound (141 mg) (48%) as a white powder. MS m/z 398 (M+H).

### Example 65: 1-N-Methyl-4-(4-fluorophenyl)-4-(3-(3-methoxy-4-methylnaphth-1-yl)-3-oxo-2-azaprop-1-yl)piperidine.

The title compound of the following structure 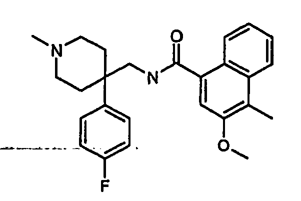 was prepared as a citrate salt, as follows. In the same manner as Example 13, but using 1-N-methyl-4-aminomethyl-4-(4-fluorophenyl)piperidine (107.5 mg, 0.484 mmol) and 3-methoxy-4-methyl-1-naphthoyl chloride (108.5 mg, 0.462 mmol), the citrate salt was isolated by filtration from Et₂O to give the title compound (217 mg) (78%) as a white powder. MS m/z 421 (M+H).

### Example 66: 1-N-Methyl-4-(4-fluorophenyl)-4-(3-(4-chloro-3-methoxynaphth-1-yl)-3-oxo-2-azaprop-1-yl)piperidine.

The title compound of the following structure 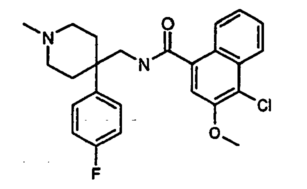 was prepared as a citrate salt, as follows. In the same manner as Example 13, but using 1-N-methyl-4-aminomethyl-4-(4-fluorophenyl)piperidine (107 mg, 0.48 mmol) and 4-chloro-3-methoxy-1-naphthoyl chloride (115.5 mg, 0.453 mmol), the citrate salt was isolated by filtration from Et₂O to give the title compound (187 mg) (67%) as a white powder. MS m/z 441 (M+H).

### Example 67: 1-N-Methyl-4-(4-fluorophenyl)-4-(3-(3-cyano-4-methylnaphth-1-yl)-3-oxo-2-azaprop-1-yl)piperidine.

The title compound of the following structure 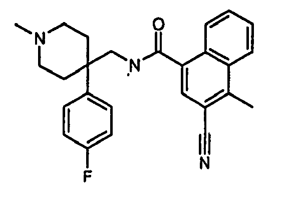 was prepared as a citrate salt, as follows. In the same manner as Example 13, but using 1-N-methyl-4-aminomethyl-4-(4-fluorophenyl)piperidine (122 mg, 0.549 mmol) and 3-cyano-4-methyl-1-naphthoyl chloride (110.9 mg, 0.483 mmol), the citrate salt was isolated by filtration from Et₂O to give the title compound (224 mg) (76%) as a white powder. MS m/z 416 (M+H).

### Example 68: 1-N-Methyl-4-(4-fluorophenyl)-4-(3-(3-cyano-4-methylnaphth-1-yl)-(3-oxo-2-N-methyl-2-azaprop-1-yl))piperidine.

The title compound of the following structure 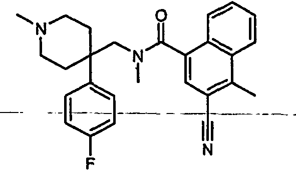 was prepared as a citrate salt, as follows. In the same manner as Example 13, but using 1-N-methyl-4-(methylaminomethyl)-4-(4-fluorophenyl)piperidine (132 mg, 0.558 mmol) and 3-cyano-4-methyl-1-naphthoyl chloride (108 mg, 0.47 mmol), the citrate salt was isolated by filtration from Et₂O to give the title compound (250 mg) (88%) as a white powder. MS m/z 430 (M+H).

### Example 69: 1-N-Methyl-4-(4-fluorophenyl)-4-(3-(4-bromonaphth-1-yl)-3-oxo-2-azaprop-1-yl)piperidine.

The title compound of the following structure 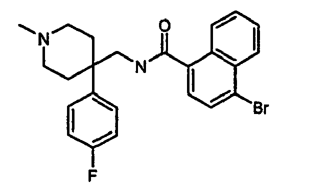 was prepared as follows. In the same manner as Example 13, but using 1-N-methyl-4-aminomethyl-4-(4-fluorophenyl)piperidine (174.6 mg, 0.785 mmol) and 4-bromo-1-naphthoyl chloride (177 mg, 0.656 mmol), the title compound (214 mg) (71 %) was obtained as a white powder. MS m/z 455 (M+H).

### Example 70: 1-N-Methyl-4-(4-fluorophenyl)-4-(3-(4-bromonaphth-1-yl)-(3-oxo-2-N-methyl-2-azaprop-1-yl))piperidine.

The title compound of the following structure 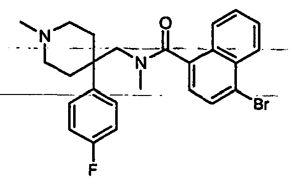 was prepared as follows. In the same manner as Example 13, but using 1-N-methyl-4-(methylaminomethyl)-4-(4-fluorophenyl)piperidine (188 mg, 0.796 mmol) and 4-bromo-1-naphthoyl chloride (179 mg, 0.664 mmol), the title compound (270.8 mg) (86%) was obtained as a white powder. MS m/z 469 (M+H).

### Examples 71 - 79:

The compounds of Examples 71 through 79 were prepared by processes similar to those given in Examples 11-14 but with replacement of 4-fluorophenyl acetonitrile with an appropriately substituted phenyl acetonitrile, compounds of Examples 71 through 79 and intermediates listed in Table 3 were obtained.

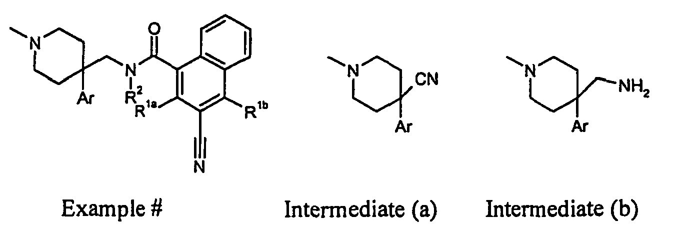

**Table 3**

| **Example #** | **Ar** | **R^{1a}** | **R²** | **R^{1b}** | **Yield (%)** | **MS m/z (M+H)** |
|---|---|---|---|---|---|---|
| 71 | 3-fluorophenyl | H | H | H | 45 | 402 |
| 71(a) | 3-fluorophenyl | | | | 97 | 219 |
| 71(b) | 3-fluorophenyl | | | | 94 | 223 |
| 72 | 3-fluorophenyl | MeO | H | H | 72 | 432 |
| 73 | 3-fluorophenyl | Et | H | H | 65 | 430 |
| 74 | 3-fluorophenyl | MeO | H | MeO | 55 | 462 |
| 75 | Phenyl | H | H | H | 65 | 384 |
| 75(a) | Phenyl | | | | 93 | 201 |
| 75(b) | Phenyl | | | | 94 | 205 |
| 76 | Phenyl | MeO | H | H | 90 | 414 |
| 77 | Phenyl | Et | H | H | 64 | 412 |
| 78 | Phenyl | MeO | H | MeO | 39 | 444 |
| 79 | Phenyl | H | Me | H | 48 | 398 |

### Example 80:

Following conventional procedures well known in the pharmaceutical art, the following representative pharmaceutical dosage forms containing a compound in accord with structural diagram I may be prepared:

| TABLET | mg/tablet |
|---|---|
| Compound in accord with structural diagram I | 50.0 |
| Mannitol, USP | 223.75 |
| Croscarmellose sodium | 60 |
| Maize starch | 15 |
| Hydroxypropylmethylcellulose (HPMC), USP | 2.25 |
| Magnesium stearate | 3.0 |

| CAPSULE | mg/capsule |
|---|---|
| Compound in accord with structural diagram I | 10.0 |
| Mannitol, USP | 488.5 |
| Croscarmellose sodium | 15 |
| Magnesium stearate | 1.5 |

The pharmaceutical dosage form is administered to a patient in need thereof at a frequency depending on the patient and the precise disease condition being treated.

## Claims

1. A compound in accord with structural diagram I: 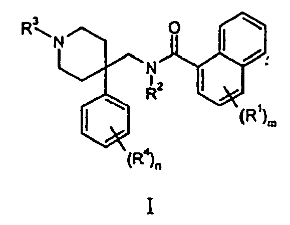 wherein:
R¹ at each occurrence is independently selected from CN, CF₃ ,OCF₃, OCHF₂, halogen, C₂₋₄alkenyl, C₂₋₄alkynyl, R^{a}, R^{b}, SR^{a}, NR^{a}R^{b}, CH₂NR^{a}R^{b}, OR^{a} or CH₂OR^{a}, where R^{a} and R^{b} are independently at each occurrence hydrogen, C₁₋₆alkyl, C(O)R^{c}, C(O)NHR^{c} or CO₂R^{c}, where R^{c} at each occurrence is C₁₋₆alkyl; or, R^{a} and R^{b} together are (CH₂)jG(CH₂)ₖ or G(CH₂)ⱼG, where G is oxygen or sulfur, j is 1, 2, 3 or 4, and k is 0, 1 or 2;
m is 1, 2 or 3 where at least one R¹ moiety is other than hydrogen;
R² and R³ are independently hydrogen, C₁₋₆alkyl or C₁₋₆alkyl substituted with C₁₋₄alkoxy;
R⁴ at each occurrence is independently selected from hydrogen, CN, CF₃ OCF₃, OCHF₂, halogen, C₁₋₄alkyl, C₂₋₄alkenyl, C₂₋₄alkynyl, SR^{a}, NR^{a}R^{b}, CH₂NR^{a}R^{b}, OR^{a} or CH₂OR^{a}, where R^{a} and R^{b} are independently at each occurrence hydrogen, C₁₋₆alkyl, C(O)R^{c}, C(O)NHR^{c} or CO₂R^{c} where R^{c} at each occurrence is C₁₋₆alkyl; or, R^{a} and R^{b} together are (CH₂)jG(CH₂)k or G(CH₂)ⱼG, and
n is 0, 1, 2 or 3;
and pharmaceutically-acceptable salts thereof

2. A compound according to Claim 1, wherein:
R¹ independently at each occurrence is CN, C₁₋₆alkyl or OR^{c} and m is 1, 2 or 3;
R² and R³ are independently hydrogen or C₁₋₆alkyl, and
R⁴ independently at each occurrence is halogen where n is 1 or 2;
and pharmaceutically-acceptable salts thereof.

3. A compound according to Claim 1 wherein:
R¹ independently at each occurrence is CN, ethyl or methoxy and m is 1, 2 or 3;
R² and R³ are independently hydrogen or methyl, and
R⁴ independently at each occurrence is halogen where n is 1 or 2;
and pharmaceutically-acceptable salts thereof.

4. A compound according to Claim 1, according to structural diagram II 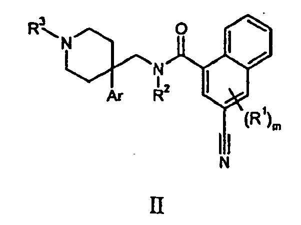 wherein Ar is selected from phenyl, 3,4-dichlorophenyl, 3-fluorophenyl, 4-fluorophenyl 3,4-difluorophenyl, 4-methoxyphenyl, 3,4-dimethoxyphenyl, 3,4-methylenedioxyphenyl, 4-difluoromethoxyphenyl or 4-trifluoromethoxyphenyl;
R¹ is selected from H, methyl, ethyl or methoxy where m is 1 or 2, and
R² and R³ are independently is selected from H or methyl, and
and pharmaceutically-acceptable salts thereof.

5. A pharmaceutically-acceptable salts of a compound according to Claim 1 made with an inorganic or organic acid which affords a physiologically-acceptable anion.

6. A pharmaceutically-acceptable salts of a compound according to Claim 5, wherein said inorganic or organic acid is selected from hydrochloric, hydrobromic, sulfuric, phosphoric, methanesulfonic, sulfamic, para-toluenesulfonic, acetic, citric, lactic, tartaric, malonic, fumaric, ethanesulfonic, benzenesulfonic, cyclohexylsulfamic, salicyclic and quinic acids.

7. A pharmaceutical composition comprising a compound according to Claim 1, pharmaceutically-acceptable salt thereof and a pharmaceutically-acceptable carrier.

8. A compound according to Claim 1 or a pharmaceutically-acceptable salt thereof for use as a medicament.

9. A compound according to Claim 8 or a pharmaceutically-acceptable salt thereof wherein the medicament is for treating a disorder or condition selected from hypertension, depression in cancer patients, depression in Parkinson's patients, postmyocardial infarction depression, subsyndromal symptomatic depression, depression in infertile women, pediatric depression, major depression, single episode depression, recurrent depression, child abuse induced depression, post partum depression, generalized anxiety disorder, agoraphobia, social phobia, simple phobias, posttraumatic stress syndrome, avoidant personality disorder, premature ejaculation, anorexia nervosa, bulimia nervosa, obesity, addictions to alcohol, cocaine, heroin, phenobarbital, nicotine or benzodiazepines; cluster headache, migraine, pain, Alzheimer's disease, obsessive-compulsive disorder, panic disorder, dementia, amnestic disorders, age-related cognitive decline, dementia in Parkinson's disease, neuroleptic-induced parkinsonism, tardive dyskinesias, hyperprolactinaemia, vasospasm, cerebral vasculature vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania and headache associated with vascular disorders in a mammal, comprising administering an effective amount of a compound according to Claim 1 or a pharmaceutically-acceptable salt thereof effective in treating such disorder or condition and a pharmaceutically-acceptable carrier.

10. A composition comprising a compound according to Claim 1 or a pharmaceutically-acceptable salt thereof, and a pharmaceutically acceptable carrier, for use in treating a disorder or condition selected from hypertension, depression in cancer patients, depression in Parkinson's patients, postmyocardial infarction depression, subsyndromal symptomatic depression, depression in infertile women, pediatric depression, major depression, single episode depression, recurrent depression, child abuse induced depression, post partum depression, generalized anxiety disorder, agoraphobia, social phobia, simple phobias, posttraumatic stress syndrome, avoidant personality disorder, premature ejaculation, anorexia nervosa, bulimia nervosa, obesity, addictions to alcohol, cocaine, heroin, phenobarbital, nicotine or benzodiazepines; cluster headache, migraine, pain, Alzheimer's disease, obsessive-compulsive disorder, panic disorder, dementia, amnestic disorders, age-related cognitive decline, dementia in Parkinson's disease, neuroleptic-induced parkinsonism, tardive dyskinesias, hyperprolactinaemia, vasospasm, cerebral vasculature vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania and headache associated with vascular disorders in a mammal.

11. The use of a compound according to Claim 1, for the preparation of a medicament useful for the treatment of hypertension, depression in cancer patients, depression in Parkinson's patients, postmyocardial infarction depression, subsyndromal symptomatic depression, depression in infertile women, pediatric depression, major depression, single episode depression, recurrent depression, child abuse induced depression, post partum depression, generalized anxiety disorder, agoraphobia, social phobia, simple phobias, posttraumatic stress syndrome, avoidant personality disorder, premature ejaculation, anorexia nervosa, bulimia nervosa, obesity, addictions to alcohol, cocaine, heroin, phenobarbital, nicotine or benzodiazepines; cluster headache, migraine, pain, Alzheimer's disease, obsessive-compulsive disorder, panic disorder, dementia, amnestic disorders, age-related cognitive decline, dementia in Parkinson's disease, neuroleptic-induced parkinsonism, tardive dyskinesias, hyperprolactinaemia, vasospasm, cerebral vasculature vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania and headache associated with vascular disorders in a mammal.

## Patentansprüche

1. Verbindung gemäß Formelbild I: 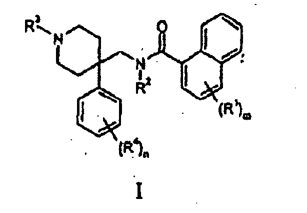 worin:
R¹ jeweils unabhängig voneinander unter CN, CF₃, OCF₃, OCHF₂, Halogen, C₂₋₄-Alkenyl, C₂₋₄-Alkinyl, R^{a}, R^{b}, SR^{a}, NR^{a}R^{b}, CH₂NR^{a}R^{b}, OR^{a} oder CH₂OR^{a} ausgewählt ist, wobei R^{a} und R^{b} jeweils unabhängig voneinander für Wasserstoff, C₁₋₆-Alkyl, C(O)R^{c}, C(O)NHR^{c} oder CO₂R_{c}, stehen, wobei R^{c} jeweils für C₁₋₆-Alkyl steht; oder R^{a} und R^{b} gemeinsam für (CH₂)ⱼG(CH₂)ₖ oder G(CH₂)ⱼG stehen, wobei G für Sauerstoff oder Schwefel steht, j für 1, 2, 3 oder 4 steht und k für 0, 1 oder 2 steht;
m für 1, 2 oder 3 steht, wobei mindestens eine Gruppe R¹ von Wasserstoff verschieden ist;
R² und R³ unabhängig voneinander für Wasserstoff, C₁₋₆-Alkyl oder durch C₁₋₄-Alkoxy substituiertes C₁₋₆-Alkyl stehen;
R⁴ jeweils unabhängig voneinander unter Wasserstoff, CN, CF₃, OCF₃, OCHF₂, Halogen, C₁₋₄-Alkyl, C₂₋₄-Alkenyl, C₂₋₄-Alkinyl, SR^{a}, NR^{a}R^{b}, CH₂NR^{a}R^{b}, OR^{a} oder CH₂OR^{a} ausgewählt ist, wobei R^{a} und R^{b} jeweils unabhängig voneinander für Wasserstoff, C₁₋₆-Alkyl, C(O)R^{c}, C(O)NHR^{c} oder CO₂R_{c} stehen, wobei R^{c} jeweils für C₁₋₆-Alkyl steht; oder R^{a} und R^{b} gemeinsam für (CH₂)ⱼG(CH₂)ₖ oder G(CH₂)ⱼG stehen, und
n für 0, 1, 2 oder 3 steht;
und pharmazeutisch annehmbare Salze davon.

2. Verbindung nach Anspruch 1, worin:
R¹ jeweils unabhängig voneinander für CN, C₁₋₆-Alkyl oder OR^{c} steht und m für 1, 2 oder 3 steht;
R² und R³ unabhängig voneinander für Wasserstoff oder C₁₋₆-Alkyl stehen und
R⁴ jeweils unabhängig voneinander für Halogen steht, wobei n für 1 oder 2 steht;
und pharmazeutisch annehmbare Salze davon.

3. Verbindung nach Anspruch 1, worin:
R¹ jeweils unabhängig voneinander für CN, Ethyl oder Methoxy steht und m für 1, 2 oder 3 steht;
R² und R³ unabhängig voneinander für Wasserstoff oder Methyl stehen und
R⁴ jeweils unabhängig voneinander für Halogen steht, wobei n für 1 oder 2 steht;
und pharmazeutisch annehmbare Salze davon.

4. Verbindung nach Anspruch 1 gemäß Formelbild II 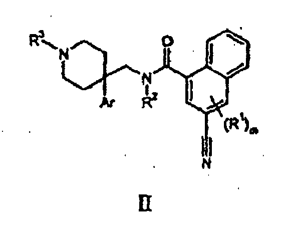 worin Ar unter Phenyl, 3,4-Dichlorphenyl, 3-Fluorphenyl, 4-Fluorphenyl, 3,4-Difluorphenyl, 4-Methoxyphenyl, 3,4-Dimethoxyphenyl, 3,4-Methylendioxyphenyl, 4-Difluormethoxyphenyl oder 4-Trifluormethoxyphenyl ausgewählt ist;
R¹ unter H, Methyl, Ethyl oder Methoxy ausgewählt ist, wo m für 1 oder 2 steht, und
R² und R³ unabhängig voneinander unter H oder Methyl ausgewählt sind,
und pharmazeutisch annehmbare Salze davon.

5. Pharmazeutisch annehmbare Salze einer Verbindung nach Anspruch 1, hergestellt mit einer anorganischen oder organischen Säure, die ein physiologisch annehmbares Anion liefert.

6. Pharmazeutisch annehmbare Salze einer Verbindung nach Anspruch 5, worin die anorganische oder organische Säure unter Salzsäure, Bromwasserstoffsäure, Schwefelsäure, Phosphorsäure, Methansulfonsäure, Sulfamidsäure, para-Toluolsulfonsäure, Essigsäure, Citronensäure, Milchsäure, Weinsäure, Malonsäure, Fumarsäure, Ethansulfonsäure, Benzolsulfonsäure, Cyclohexylsulfamidsäure, Salicylsäure und Chinasäure ausgewählt ist.

7. Pharmazeutische Zusammensetzung, enthaltend eine Verbindung nach Anspruch 1 oder ein pharmazeutisch annehmbares Salz davon und einen pharmazeutisch annehmbaren Träger.

8. Verbindung nach Anspruch 1 oder ein pharmazeutisch annehmbares Salz davon zur Verwendung als Arzneimittel.

9. Verbindung nach Anspruch 8 oder ein pharmazeutisch annehmbares Salz davon, wobei das Arzneimittel zur Behandlung einer unter Hypertonie, Depression bei Krebspatienten, Depression bei Parkinson-Patienten, Depression nach Myokardinfarkt, subsyndromaler symptomatischer Depression, Depression bei unfruchtbaren Frauen, Depression bei Kindern, Major Depression, einepisodiger Depression, wiederkehrender Depression, durch Kindesmißbrauch induzierter Depression, postpartaler Depression, generalisierter Angstneurose, Agoraphobie, sozialer Phobie, einfachen Phobien, posttraumatischem Streßsyndrom, ängstlich-vermeidender Persönlichkeitsstörung, vorzeitiger Ejakulation, Anorexia nervosa, Bulimia nervosa, Fettleibigkeit, Abhängigkeit von Alkohol, Kokain, Heroin, Phenobarbital, Nicotin oder Benzodiazepinen; Cluster-Kopfschmerz, Migräne, Schmerzen, Alzheimer-Krankheit, Zwangsneurose, Panikstörung, Demenz, amnestischen Störungen, altersbedingter kognitiver Verschlechterung, Demenz bei Parkinsonscher Krankheit, Neuroleptika-induziertem Parkinsonismus, tardiven Dyskinesien, Hyperprolactinämie, Vasospasmus, Hirngefäß-Vasospasmus, zerebellärer Ataxie, Störungen des Magen-Darm-Trakts, negativen Symptomen von Schizophrenie, prämenstruellem Syndrom, Fibromyalgiesyndrom, Streßinkontinenz, Tourette-Syndrom, Trichotillomanie, Kleptomanie, Impotenz des Mannes, Aufmerksamkeitsdefizit-Hyperaktivitätssyndrom, chronischer paroxysmaler Hemikranie und mit Gefäßstörungen assoziiertem Kopfschmerz ausgewählten Störung oder Beschwerde bei einem Säugetier vorgesehen ist, wobei man eine wirksame Menge einer Verbindung nach Anspruch 1 oder ein pharmazeutisch annehmbares Salz davon, das bei der Behandlung einer derartigen Störung oder Beschwerde wirksam ist, und einen pharmazeutisch annehmbaren Träger verabreicht.

10. Zusammensetzung, enthaltend eine Verbindung nach Anspruch 1 oder ein pharmazeutisch annehmbares Salz davon und einen pharmazeutisch annehmbaren Träger, zur Verwendung bei der Behandlung einer unter Hypertonie, Depression bei Krebspatienten, Depression bei Parkinson-Patienten, Depression nach Myokardinfarkt, subsyndromaler symptomatischer Depression, Depression bei unfruchtbaren Frauen, Depression bei Kindern, Major Depression, einepisodiger Depression, wiederkehrender Depression, durch Kindesmißbrauch induzierter Depression, postpartaler Depression, generalisierter Angstneurose, Agoraphobie, sozialer Phobie, einfachen Phobien, posttraumatischem Streßsyndrom, ängstlich-vermeidender Persönlichkeitsstörung, vorzeitiger Ejakulation, Anorexia nervosa, Bulimia nervosa, Fettleibigkeit, Abhängigkeit von Alkohol, Kokain, Heroin, Phenobarbital, Nicotin oder Benzodiazepinen; Cluster-Kopfschmerz, Migräne, Schmerzen, Alzheimer-Krankheit, Zwangsneurose, Panikstörung, Demenz, amnestischen Störungen, altersbedingter kognitiver Verschlechterung, Demenz bei Parkinsonscher Krankheit, Neuroleptika-induziertem Parkinsonismus, tardiven Dyskinesien, Hyperprolactinämie, Vasospasmus, Hirngefäß-Vasospasmus, zerebellärer Ataxie, Störungen des Magen-Darm-Trakts, negativen Symptomen von Schizophrenie, prämenstruellem Syndrom, Fibromyalgiesyndrom, Streßinkontinenz, Tourette-Syndrom, Trichotillomanie, Kleptomanie, Impotenz des Mannes, Aufmerksamkeitsdefizit-Hyperaktivitätssyndrom, chronischer paroxysmaler Hemikranie und mit Gefäßstörungen assoziiertem Kopfschmerz ausgewählten Störung oder Beschwerde bei einem Säugetier.

11. Verwendung einer Verbindung nach Anspruch 1 zur Herstellung eines Arzneimittels, das zur Verwendung bei der Behandlung von Hypertonie, Depression bei Krebspatienten, Depression bei Parkinson-Patienten, Depression nach Myokardinfarkt, subsyndromaler symptomatischer Depression, Depression bei unfruchtbaren Frauen, Depression bei Kindern, Major Depression, einepisodiger Depression, wiederkehrender Depression, durch Kindesmißbrauch induzierter Depression, postpartaler Depression, generalisierter Angstneurose, Agoraphobie, sozialer Phobie, einfachen Phobien, posttraumatischem Streßsyndrom, ängstlich-vermeidender Persönlichkeitsstörung, vorzeitiger Ejakulation, Anorexia nervosa, Bulimia nervosa, Fettleibigkeit, Abhängigkeit von Alkohol, Kokain, Heroin, Phenobarbital, Nicotin oder Benzodiazepinen; Cluster-Kopfschmerz, Migräne, Schmerzen, Alzheimer-Krankheit, Zwangsneurose, Panikstörung, Demenz, amnestischen Störungen, altersbedingter kognitiver Verschlechterung, Demenz bei Parkinsonscher Krankheit, Neuroleptika-induziertem Parkinsonismus, tardiven Dyskinesien, Hyperprolactinämie, Vasospasmus, Hirngefäß-Vasospasmus, zerebellärer Ataxie, Störungen des Magen-Darm-Trakts, negativen Symptomen von Schizophrenie, prämenstruellem Syndrom, Fibromyalgiesyndrom, Streßinkontinenz, Tourette-Syndrom, Trichotillomanie, Kleptomanie, Impotenz des Mannes, Aufmerksamkeitsdefizit-Hyperaktivitätssyndrom, chronischer paroxysmaler Hemikranie und mit Gefäßstörungen assoziiertem Kopfschmerz ausgewählten Störung oder Beschwerde bei einem Säugetier geeignet ist.

## Revendications

1. Composé selon le schéma structural I : 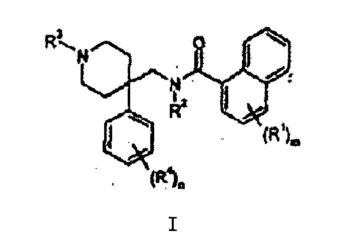 dans lequel :
R¹ à chaque occurrence est indépendamment choisi parmi CN, CF₃, OCF₃, OCHF₂, un halogène, un alcényle en C₂₋₄, un alcynyle en C₂₋₄, R^{a}, R^{b} SR^{a}, NR^{a}R^{b}, CH₂NR^{a}R^{b}, OR^{a} ou CH₂OR^{a}, où R^{a} et R^{b} sont indépendamment à chaque occurrence un atome d'hydrogène, un alkyle en C₁₋₆, C(O)R^{c}, C(O)NHR^{c} ou CO₂R^{c}, où R^{c} à chaque occurrence est un alkyle en C₁₋₆ ; ou R^{a} et R^{b} sont conjointement (CH₂)ⱼG(CH₂)ₖ ou G(CH₂)ⱼG, où G est un atome d'oxygène ou de soufre, j est 1, 2, 3 ou 4, et k est 0, 1 ou 2 ;
m est 1, 2 ou 3 où au moins un fragment R¹ est autre qu'un atome d'hydrogène ;
R² et R³ sont indépendamment un atome d'hydrogène, un alkyle en C₁₋₆ ou un alkyle en C₁₋₆ substitué par un alcoxy en C₁₋₄ ;
R⁴ à chaque occurrence est indépendamment choisi parmi un atome d'hydrogène, CN, CF₃, OCF₃, OCHF₂, un halogène, un alkyle en C₁₋₄, un alcényle en C₂₋₄, un alcynyle en C₂₋₄, SR^{a}, NR^{a}R^{b}, CH₂NR^{a}R^{b}, OR^{a} ou CH₂OR^{a}, où R^{a} et R^{b} sont indépendamment à chaque occurrence un atome d'hydrogène, un alkyle en C₁₋₆, C(O)R^{c}, C(O)NHR^{c} ou CO₂R^{c}, où R^{c} à chaque occurrence est un alkyle en C₁₋₆ ; ou, R^{a} et R^{b} sont conjointement (CH₂)ⱼG(CH₂)ₖ ou G(CH₂₎ⱼG, et
n est 0, 1, 2 ou 3 ;
et des sels pharmaceutiquement acceptables de celui-ci.

2. Composé selon la revendication 1, dans lequel :
R¹ indépendamment à chaque occurrence est CN, un alkyle en C₁₋₆ ou OR^{c} et m est 1, 2 ou 3 ;
R² et R³ sont indépendamment un atome d'hydrogène ou un alkyle en C₁₋₆, et
R⁴ indépendamment à chaque occurrence est un halogène où n est 1 ou 2 ;
et des sels pharmaceutiquement acceptables de celui-ci.

3. Composé selon la revendication 1, dans lequel :
R¹ indépendamment à chaque occurrence est CN, un éthyle ou un méthoxy et m est 1, 2 ou 3 ;
R² et R³ sont indépendamment un atome d'hydrogène ou un méthyle, et
R⁴ indépendamment à chaque occurrence est un halogène où n est 1 ou 2 ;
et des sels pharmaceutiquement acceptables de celui-ci.

4. Composé selon la revendication 1, selon le schéma structural II 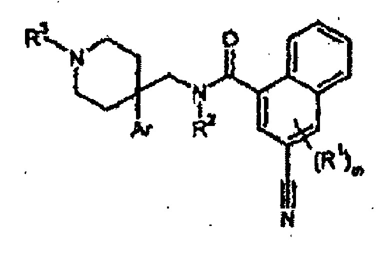 dans lequel Ar est choisi parmi un phényle, un 3,4-dichlorophényle, un 3-fluorophényle, un 4-fluorophényle, un 3,4-difluorophényle, un 4-méthoxyphényle, un 3,4-diméthoxyphényle, un 3,4-méthylènedioxyphényle, un 4-difluorométhoxyphényle ou un 4-trifluorométhoxyphényle ;
R¹ est choisi parmi H, un méthyle, un éthyle ou un méthoxy où m est 1 ou 2, et
R² et R³ sont indépendamment choisis parmi H ou un méthyle,
et des sels pharmaceutiquement acceptables de celui-ci.

5. Sels pharmaceutiquement acceptables d'un composé selon la revendication 1 préparés avec un acide organique ou inorganique qui produit un anion physiologiquement acceptable.

6. Sels pharmaceutiquement acceptables d'un composé selon la revendication 5, **caractérisés en ce que** ledit acide organique ou inorganique est choisi parmi les acides chlorhydrique, bromhydrique, sulfurique, phosphorique, méthanesulfonique, sulfamique, para-toluènesulfonique, acétique, citrique, lactique, tartrique, malonique, fumarique, éthanesulfonique, benzènesulfonique, cyclohexylsulfamique, salicylique et quinique.

7. Composition pharmaceutique comprenant un composé selon la revendication 1, un sel pharmaceutiquement acceptable de celui-ci et un véhicule pharmaceutiquement acceptable.

8. Composé selon la revendication 1 ou un sel pharmaceutiquement acceptable de celui-ci pour une utilisation en tant que médicament.

9. Composé selon la revendication 8 ou un sel pharmaceutiquement acceptable de celui-ci **caractérisé en ce que** le médicament est destiné à traiter un trouble ou état choisi parmi l'hypertension, la dépression chez les patients cancéreux, la dépression chez les patients parkinsoniens, la dépression post-infarctus du myocarde, la dépression symptomatique subsyndromique, la dépression chez les femmes stériles, la dépression pédiatrique, la dépression majeure, la dépression à épisode unique, la dépression récurrente, la dépression induite par des mauvais traitements infligés à un enfant, la dépression post-partum, un trouble d'anxiété généralisée, l'agoraphobie, la phobie sociale, les phobies simples, le syndrome de stress post-traumatique, les troubles de la personnalité avec évitement, l'éjaculation précoce, l'anorexie mentale, la boulimie mentale, l'obésité, les dépendances à l'alcool, la cocaïne, l'héroïne, le phénobarbital, la nicotine ou les benzodiazépines ; l'algie vasculaire de la face, la migraine, la douleur, la maladie d'Alzheimer, les troubles obsessionnels compulsifs, les troubles paniques, la démence, les troubles amnésiques, le déclin cognitif associé au vieillissement, la démence dans la maladie de Parkinson, le parkinsonisme induit par les neuroleptiques, les dyskinésies tardives, l'hyperprolactinémie, les angiospasmes, les angiospasmes cérébrovasculaires, l'ataxie cérébelleuse, les troubles du tractus gastro-intestinal, les symptômes négatifs de la schizophrénie, le syndrome prémenstruel, le syndrome de fibromyalgie, l'incontinence due au stress, le syndrome de Tourette, la trichotillomanie, la kleptomanie, l'impuissance masculine, les troubles d'hyperactivité due à un déficit de l'attention, l'hémicranie paroxystique et les maux de tête associés à des troubles vasculaires chez un mammifère, comprenant l'administration d'une quantité efficace d'un composé selon la revendication 1 ou d'un sel pharmaceutiquement acceptable de celui-ci efficace dans le traitement de tels troubles ou états et un véhicule pharmaceutiquement acceptable.

10. Composition comprenant un composé selon la revendication 1 ou un sel pharmaceutiquement acceptable de celui-ci, et un véhicule pharmaceutiquement acceptable, pour une utilisation dans le traitement d'un trouble ou état choisi parmi l'hypertension, la dépression chez les patients cancéreux, la dépression chez les patients parkinsoniens, la dépression post-infarctus du myocarde, la dépression symptomatique subsyndromique, la dépression chez les femmes stériles, la dépression pédiatrique, la dépression majeure, 1 la dépression à épisode unique, la dépression récurrente, la dépression induite par des mauvais traitements infligés à un enfant, la dépression post-partum, un trouble d'anxiété généralisée, l'agoraphobie, la phobie sociale, les phobies simples, le syndrome de stress post-traumatique, les troubles de la personnalité avec évitement, l'éjaculation précoce, l'anorexie mentale, la boulimie mentale, l'obésité, les dépendances à l'alcool, la cocaïne, l'héroïne, le phénobarbital, la nicotine ou les benzodiazépines ; l'algie vasculaire de la face, la migraine, la douleur, la maladie d'Alzheimer, les troubles obsessionnels compulsifs, les troubles paniques, la démence, les troubles amnésiques, le déclin cognitif associé au vieillissement, la démence dans la maladie de Parkinson, le parkinsonisme induit par les neuroleptiques, les dyskinésies tardives, l'hyperprolactinémie, les angiospasmes, les angiospasmes cérébrovasculaires, l'ataxie cérébelleuse, les troubles du tractus gastro-intestinal, les symptômes négatifs de la schizophrénie, le syndrome prémenstruel, le syndrome de fibromyalgie, l'incontinence due au stress, le syndrome de Tourette, la trichotillomanie, la kleptomanie, l'impuissance masculine, les troubles d'hyperactivité due à un déficit de l'attention, l'hémicranie paroxystique et les maux de tête associés à des troubles vasculaires chez un mammifère.

11. Utilisation d'un composé selon la revendication 1, pour la préparation d'un médicament utile pour le traitement de l'hypertension, la dépression chez les patients cancéreux, la dépression chez les patients parkinsoniens, la dépression post-infarctus du myocarde, la dépression symptomatique subsyndromique, la dépression chez les femmes stériles, la dépression pédiatrique, la dépression majeure, la dépression à épisode unique, la dépression récurrente, la dépression induite par des mauvais traitements infligés à un enfant, la dépression post-partum, un trouble d'anxiété généralisée, l'agoraphobie, la phobie sociale, les phobies simples, le syndrome de stress post-traumatique, les troubles de la personnalité avec évitement, l'éjaculation précoce, l'anorexie mentale, la boulimie mentale, l'obésité, les dépendances à l'alcool, la cocaïne, l'héroïne, le phénobarbital, la nicotine ou les benzodiazépines ; l'algie vasculaire de la face, la migraine, la douleur, la maladie d'Alzheimer, les troubles obsessionnels compulsifs, les troubles paniques, la démence, les troubles amnésiques, le déclin cognitif associé au vieillissement, la démence dans la maladie de Parkinson, le parkinsonisme induit par les neuroleptiques, les dyskinésies tardives, l'hyperprolactinémie, les angiospasmes, les angiospasmes cérébrovasculaires, l'ataxie cérébelleuse, les troubles du tractus gastro-intestinal, les symptômes négatifs de la schizophrénie, le syndrome prémenstruel, le syndrome de fibromyalgie, l'incontinence due au stress, le syndrome de Tourette, la trichotillomanie, la kleptomanie, l'impuissance masculine, les troubles d'hyperactivité due à un déficit de l'attention, l'hémicranie paroxystique et les maux de tête associés à des troubles vasculaires chez un mammifère.
